(19) Europäisches Patentamt
European Patent Office
Office européen des brevets

(11) EP 1 382 329 A1

(12) **EUROPÄISCHE PATENTANMELDUNG**

(43) Veröffentlichungstag:
**21.01.2004 Patentblatt 2004/04**

(21) Anmeldenummer: **03013429.0**

(22) Anmeldetag: **23.06.2003**

(51) Int Cl.$^7$: **A61K 7/48**, A61K 7/42, A61K 31/352, A61K 31/7048, A61P 17/00, A61P 39/06, A23L 1/30

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**AL LT LV MK**

(30) Priorität: **18.07.2002 DE 10232595**

(71) Anmelder: **MERCK PATENT GmbH**
**64293 Darmstadt (DE)**

(72) Erfinder:
- **Buchholz, Herwig, Dr.**
  **60599 Frankfurt (DE)**
- **Carola, Christophe, Dr.**
  **63225 Langen (DE)**
- **Perruchon, Sophie**
  **67310 Wasselonne (FR)**

(54) **Lichtschutzmittel enthaltend ein Flavonoid**

(57) Die Erfindung betrifft eine Zubereitung mit Lichtschutzeigenschaften, enthaltend zumindest eine Verbindung der Formel I

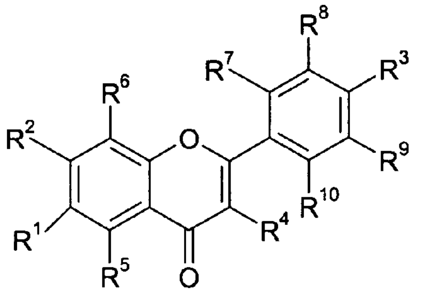

I

wobei $R^1$ und $R^2$ ausgewählt sind aus H, und $OR^{11}$, wobei $OR^{11}$ unabhängig voneinander steht für OH, geradkettige oder verzweigte $C_1$- bis $C_{20}$-Alkyloxygruppen, geradkettigen oder verzweigten $C_3$- bis $C_{20}$-Alkenyloxygruppen, geradkettigen oder verzweigten $C_1$- bis $C_{20}$-Hydroxyalkoxygruppen, wobei die Hydroxygruppe(n) an ein primäre oder sekundäre Kohlenstoffatome der Kette gebunden sein können und weiter die Alkylkette auch durch Sauerstoff unterbrochen sein kann, und/oder $C_3$- bis $C_{10}$-Cycloalkyloxygruppen und/oder $C_3$- bis $C_{12}$-Cycloalkenyloxygruppen, wobei die Ringe jeweils auch durch-$(CH_2)_n$-Gruppen mit n = 1 bis 3 überbrückt sein können und/oder, Mono- und/oder Oligoglycosylreste, mit der Maßgabe, dass mindestens ein Rest aus $R^1$ und $R^2$ steht für $OR^{11}$, und $R^3$ steht für einen Rest $OR^{11}$ und $R^4$ bis $R^7$ und $R^{10}$ gleich oder verschieden sein können, und unabhängig voneinander stehen für bezüglich der UV-Eigenschaften weitgehend inerte Reste.

EP 1 382 329 A1

**Beschreibung**

Die vorliegende Erfindung betrifft Zubereitung mit Lichtschutzeigenschaften, deren Herstellung und Verwendung.

Eine mehr oder minder stark ausgeprägte Sonnenbräune der Haut gilt in der modernen Gesellschaft als attraktiv und als Ausdruck von Dynamik und Sportlichkeit. Neben dieser erwünschten Wirkung der Sonne auf die Haut treten eine Reihe von unerwünschten Nebenwirkungen auf, wie Sonnenbrand oder vorzeitige Hautalterung und Faltenbildung. Inzwischen sind eine Anzahl von leistungsfähigen UV-Filtern entwickelt worden, die in Form von Cremes, Lotionen oder Gelen auf die Haut aufgetragen auch bei stärkerer Sonneneinwirkung die Entwicklung von Sonnenbrand wirksam verhindern können. Der in der pharmazeutischen oder kosmetischen Zubereitung enthaltene UV-Filter bildet auf der Oberfläche der Haut einen Film bzw. eine Schicht aus und dringt nicht mit weiteren in der Zubereitung enthaltenen pflegenden Substanzen in tiefere Hautschichten vor. Bekannte UV-Filter bzw. Sonnenschutzmittel wirken also nur in der Weise, dass sie bestimmte Bereiche des Sonnenlichts absorbieren und somit diese Strahlung nicht in tiefere Schichten der Haut vordringen kann. Bekanntlich wird der gefährlichste Teil der Sonnenstrahlung von den ultravioletten Strahlen mit einer Wellenlänge von weniger als 400 nm gebildet. Die untere Grenze der ultravioletten Strahlen, welche die Erdoberfläche erreichen, ist durch die Absorption in der Ozonschicht auf ca. 280 nm beschränkt. Die heute in der Kosmetik üblichen Sonnenschutzfilter absorbieren in einem Wellenlängenbereich von 280 bis 400 nm. Dieser Bereich umfaßt UV-B-Strahlen mit einer Wellenlänge zwischen 280 und 320 nm, die bei der Bildung eines Sonnenerythems eine entscheidende Rollen spielen, wie auch UV-A-Strahlen, mit einer Wellenlänge zwischen 320 und 400 nm, welche die Haut bräunen aber auch altern lassen, die Auslösung einer erythematösen Reaktion begünstigen oder diese Reaktion bei bestimmten Menschen vergrößern oder sogar phototoxische oder photoallergische und irritative Reaktionen auslösen können.

Hautschädigungen werden nicht nur durch Sonnenlicht verursacht, sondern auch durch andere äußere Einflüsse, wie Kälte oder Wärme. Ferner unterliegt die Haut einer natürlichen Alterung, wodurch Falten entstehen und die Spannkraft der Haut nachläßt.

Aufgabe pflegender Kosmetik ist es, nach Möglichkeit den Eindruck einer jugendlichen Haut zu erhalten. Prinzipiell stehen verschiedene Wege offen, um diesen Weg zu erreichen. So können bereits vorhandene Schädigungen der Haut, wie unregelmäßige Pigmentierung oder Faltenbildung, durch abdeckende Puder oder Cremes ausgeglichen werden. Ein anderer Ansatzpunkt ist, die Haut vor Umwelteinflüssen zu schützen, die zu einer dauerhaften Schädigung und damit Alterung der Haut führen. Die Idee ist also, vorbeugend einzugreifen und dadurch den Alterungsprozess hinauszuzögern. Ein Beispiel sind hierfür die bereits erwähnten UV-Filter, welche durch Absorption bestimmter Wellenlängenbereiche eine Schädigung der Haut vermeiden oder zumindest vermindern. Während bei UV-Filtern das schädigende Ereignis, die UV-Strahlung, von der Haut abgeschirmt wird, versucht man bei einem weiteren Weg, die natürlichen Abwehr- bzw. Reparaturmechanismen der Haut gegen das schädigende Ereignis zu unterstützen. Schließlich verfolgt man als weiteren Ansatzpunkt die mit zunehmendem Alter sich abschwächenden Abwehrfunktionen der Haut gegen schädigende Einflüsse auszugleichen, indem Substanzen von außen zugeführt werden, die diese nachlassende Abwehr- bzw. Reparaturfunktion ersetzen können. Beispielsweise besitzt die Haut die Fähigkeit, Radikale, die durch äußere oder innere Stressfaktoren erzeugt werden, abzufangen. Diese Fähigkeit schwächt sich mit zunehmendem Alter ab, wodurch sich der Alterungsprozess mit zunehmendem Alter beschleunigt.

Eine weitere Schwierigkeit bei der Herstellung von Kosmetika besteht darin, dass Wirkstoffe, die in kosmetische Zubereitungen eingearbeitet werden sollen, oftmals nicht stabil sind und in der Zubereitung geschädigt werden können. Die Schädigungen können beispielsweise durch eine Reaktion mit Luftsauerstoff oder durch die Absorption von UV-Strahlen verursacht werden. Die so geschädigten Moleküle können durch ihre Strukturänderung z.B. ihre Farbe ändern und/oder ihre Wirksamkeit verlieren.

In der neueren Literatur gibt es verschiedene Lösungsansätze für die genannten Probleme:

In der DE 197 46 654 A1 werden photostabile UV-Filter enthaltende kosmetische und pharmazeutische Zubereitungen beschrieben. Dabei werden 4,4-Diarylbutadiene als photostabile UV-Filter in kosmetischen und pharmazeutischen Zubereitungen zum Schutz der menschlichen Haut oder menschlicher Haare gegen Sonnenstrahlen, allein oder zusammen mit an sich für kosmetische und pharmazeutische Zubereitungen bekannten, im UV-Bereich absorbierenden Verbindungen verwendet.

In der DE 195 08 608 A1 wird ein lichtbeständiges kosmetisches Mittel beschrieben. Es werden kosmetische Mittel zum Schutz vor UV-Strahlen der Wellenlänge zwischen 280 und 400 nm offenbart, die mindestens ein Tetraalkylquercetin, in einem kosmetisch annehmbaren Medium auf Ölbasis enthalten.

In der DE 197 55 504 A1 wird die Verwendung von Flavonen und Flavonoiden gegen die UV-induzierte Zersetzung von Dibenzoylmethan und dessen Derivaten beschrieben.

In der WO 02/00214 für die Verwendung von bestimmten Flavon-Derivaten zur Herstellung von oralen Arzneimitteln zur systemischen Behandlung und Prophylaxe von UV-induzierten Dermatosen, insbesondere der polymorphen Lichtdermatosen und ihren Unterformen und/oder unerwünschten Langzeitfolgen von UV-Bestrahlung, besonders der Lichtalterung, beschrieben. Bevorzugte Flavon-Derivate sind dabei insbesondere natürlich vorkommende Bioflavonoide, wie Rutin, Naringin, Naringenin, Hesperidin, Hesperetin, Taxifolin etc. sowie Derivate davon, wie Troxerutin oder Monoxerutin.

In der europäischen Patentanmeldung EP-A-1 147 764 werden kosmetische Zusammensetzung beschrieben die bis zu 10 Gew.-% Polymethoxyflavone mit mindestens vier Methoxy-funktionen enthalten. Vorteile dieser Zusammensetzung sind ein hautauffallender Effekt i. V. m. der Verhinderung von Falten, sowie Lagerstabilität und Sicherheit bei der Anwendung.

In der internationalen Patentanmeldung WO 00/61095 werden Mischungen von Polyphenolen mit Vitaminen beschrieben. Diese Mischungen eignen sich zum Einsatz in kosmetischen oder dermatologischen Zusammensetzungen und sind optimiert zum Fangen freier Radikale wie Hydroxy-Radikale oder Peroxiden. Insbesondere bevorzugt ist dabei die Kombination von Troxerutin mit $\alpha$-Tocopherolsuccinat und Ascorbyl-palmitat.

Es besteht jedoch weiterhin Bedarf nach hautverträglichen UV-Filtern, die sich auch zum Einsatz in hautpflegenden Zubereitungen eignen.

Aufgabe der Erfindung ist es daher, eine Zusammensetzung zur Verfügung zu stellen, welche eine schützende Wirkung gegen UV-Strahlen aufweist und/oder eine schützende Wirkung gegen oxidativen Stress auf Körperzellen ausübt und/oder einer Alterung der Haut entgegenwirkt.

Überraschend wurde dabei gefunden dass bestimmte Flavonoide sich hervorragend als UV-Filter eignen. Ein erster Gegenstand der vorliegenden Erfindung sind daher Zubereitungen, die diese Flavonoide enthalten.

Diese Aufgabe wird gelöst durch eine Zubereitung mit Lichtschutzeigenschaften, enthaltend zumindest eine Verbindung der Formel 1

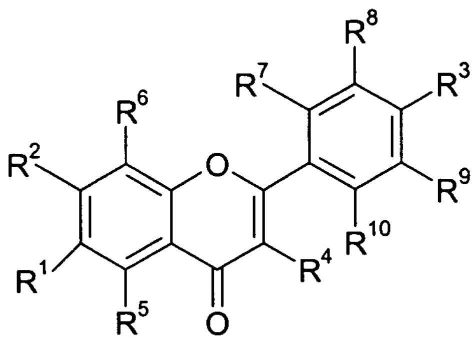

I

wobei $R^1$ und $R^2$ ausgewählt sind aus

- H
- und $OR^{11}$, wobei $OR^{11}$ unabhängig voneinander steht für
  - OH
  - geradkettige oder verzweigte $C_1$ bis $C_{20}$-Alkyloxygruppen,
  - geradkettigen oder verzweigten $C_3$- bis $C_{20}$-Alkenyloxygruppen,
  - geradkettigen oder verzweigten $C_1$- bis $C_{20}$-Hydroxyalkoxygruppen, wobei die Hydroxygruppe(n) an ein primäre oder sekundäre Kohlenstoffatome der Kette gebunden sein können und weiter die Alkylkette auch durch Sauerstoff unterbrochen sein kann, und/oder
  - $C_3$- bis $C_{10}$-Cycloalkyloxygruppen und/oder $C_3$- bis $C_{12}$-Cycloalkenyloxygruppen, wobei die Ringe jeweils auch durch $-(CH_2)_n$-Gruppen mit n = 1 bis 3 überbrückt sein können und/oder,
  - Mono- und/oder Oligoglycosylreste,

mit der Maßgabe, dass mindestens ein Rest aus $R^1$ und $R^2$ steht für $OR^{11}$,
und $R^3$ steht für einen Rest $OR^{11}$ und
$R^4$ bis $R^7$ und $R^{10}$ gleich oder verschieden sein können, und unabhängig voneinander stehen für

- H
- geradkettige oder verzweigte $C_1$- bis $C_{20}$-Alkylgruppen,
- geradkettige oder verzweigte $C_3$- bis $C_{20}$-Alkenylgruppen,
- geradkettige oder verzweigte $C_1$- bis $C_{20}$-Hydroxyalkylgruppen, wobei die Hydroxygruppe an ein primäres oder

sekundäres Kohlenstoffatom der Kette gebunden sein kann und weiter die Alkylkette auch durch Sauerstoff unterbrochen sein kann, und/oder

- $C_3$- bis $C_{10}$-Cycloalkylgruppen und/oder $C_3$- bis $C_{12}$-Cycloalkenylgruppen, wobei die Ringe jeweils auch durch $-(Ch_2)_n$-Gruppen mit n = 1 bis 3 überbrückt sein können und

$R^8$ und $R^9$ gleich oder verschieden sein können, und unabhängig voneinander stehen für

- H
- $OR^{11}$
- geradkettige oder verzweigte $C_1$- bis $C_{20}$-Alkylgruppen,
- geradkettige oder verzweigte $C_3$- bis $C_{20}$-Alkenylgruppen,
- geradkettige oder verzweigte $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder
- $C_3$- bis $C_{10}$-Cycloalkylgruppen und/oder $C_3$- bis C12-Cycloalkenylgruppen, wobei die Ringe jeweils auch durch $-(CH_2)_n$-Gruppen mit n = 1 bis 3 überbrückt sein können.

Vorteile der erfindungsgemäßen Zusammensetzungen sind dabei insbesondere die UV-Licht filternde Wirkung und die gute Hautverträglichkeit. Zusätzlich sind die hier beschriebenen Verbindungen farblos oder nur schwach gefärbt und führen so, im Gegensatz zu vielen bekannten natürlich vorkommenden Flavonoiden, nicht zu Verfärbungen der Zubereitungen.

Ein weiterer Gegenstand der vorliegenden Erfindung ist daher auch die Verwendung der Verbindungen gemäß Formel I, wie oben angegeben, als UV-Filter bzw. zur Herstellung einer Zubereitung mit Lichtschutzeigenschaften.

Bei den Zubereitungen handelt es sich dabei üblicherweise um topisch anwendbare Zubereitungen, beispielsweise kosmetische oder dermatologische Formulierungen. Die Zubereitungen enthalten in diesem Fall einen kosmetisch oder dermatologisch geeigneten Träger und je nach gewünschtem Eigenschaftsprofil optional weitere geeignete Inhaltsstoffe.

Unter den erfindungsgemäß einzusetzenden Flavonoiden der Formel I finden sich dabei Breitband-UV-Filter, die alleine oder in Kombination mit weiteren UV-Filtern eingesetzt werden können.

Andere ebenfalls bevorzugte Verbindungen der Formel I zeigen ein Absorptionsmaximum im Grenzbereich zwischen der UV-B- und der UV-A-Strahlung. Als UV-A-II-Filter ergänzen sie daher vorteilhaft das Absorptionsspektrum von handelsüblichen UV-B- bzw. UV-A-I-Filtern.

Bevorzugte erfindungsgemäße Zubereitungen mit Lichtschutzeigenschaften enthalten zumindest eine Verbindung der Formel I, wobei $R^3$ steht für

- OH oder
- geradkettige oder verzweigte $C_1$- bis $C_{20}$-Alkoxygruppen, vorzugsweise Methoxy, Ethoxy oder Ethylhexyloxy oder
- Mono- und/oder Oligoglycosylreste, vorzugsweise Glucosylreste und

$R^1$ und/oder $R^2$ vorzugsweise stehen für

- OH oder
- geradkettige oder verzweigte $C_1$- bis $C_{20}$-Alkoxygruppen, vorzugsweise Methoxy, Ethoxy oder Ethylhexyloxy oder
- Mono- und/oder Oligoglycosylreste, vorzugsweise Glucosylreste.

Diese bevorzugten Verbindungen zeichnen sich durch eine besonders intensive UV-Absorption aus.

Zusätzlich haben solche bevorzugten Verbindungen Vorteile bei der Einarbeitung in die Zubereitungen:

- Mono- und/oder Oligoglycosylreste verbessern die Wasserlöslichkeit der erfindungsgemäß einzusetzenden Verbindungen;
- geradkettige oder verzweigte $C_1$- bis $C_{20}$-Alkoxygruppen, insbesondere die langkettigen Alkoxyfunktionen, wie Ethylhexyloxy-Gruppen erhöhen die Öllöslichkeit der Verbindungen;

d.h. über die geeignete Auswahl der Substituenten kann die Hydrophilie bzw. Lipophilie der erfindungsgemäßen Verbindungen gesteuert werden.

Als Mono- oder Oligosaccharid-reste bevorzugt sind dabei Hexosylreste, insbesondere Ramnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch vorteilhaft sein, Pentosylreste zu verwenden.

Die Glycosylreste können α- oder β-glycosidisch mit dem Grundkörper verbunden sein. Ein bevorzugtes Disaccharid ist beispielsweise das 6-O-(6-deoxy-α-Lmannopyranosyl)-β-D-glucopyranosid.

Die erfindungsgemäßen Zubereitungen können in ebenfalls bevorzugten Ausführungsformen der Erfindung jedoch auch in der Zubereitungs-Matrix schlecht oder nicht lösliche Verbindungen der Formel I enthalten. In diesem Fall liegen die Verbindungen vorzugsweise in feinteiliger Form in der kosmetischen Zubereitung dispergiert vor.

Die Reste $R^4$ bis $R^7$ und $R^{10}$ beeinflussen die erwünschte molare UV-Absorption nach bisherigen Erkenntnissen nur unwesentlich und werden im Sinne der vorliegenden Erfindung daher als weitgehend inert auf die UV-Absorption angesehen.. Da bei der Zubereitung jedoch die Absorption pro Gramm Substanz wesentlich ist, sind erfindungsgemäß Zubereitung bevorzugt, die zumindest eine Verbindung der Formel I enthalten, die dadurch gekennzeichnet ist, dass $R^4$ bis $R^7$ und $R^{10}$ H sind.

Es hat sich gezeigt, dass die Intensität der UV-Absorption insbesondere dann hoch ist, wenn $R^3$ steht für geradkettige oder verzweigte $C_1$- bis $C_{20}$-Alkoxygruppen, vorzugsweise Methoxy, Ethoxy oder Ethylhexyloxy, und $R^8$ und $R^9$ gleich sind und stehen für H oder geradkettige oder verzweigte $C_1$bis $C_{20}$-Alkoxygruppen, vorzugsweise Methoxy, Ethoxy oder Ethylhexyloxy. Daher sind Zubereitungen mit Lichtschutzeigenschaften enthaltend zumindest eine Verbindung der Formel I, die dadurch gekennzeichnet ist, dass $R^3$ steht für geradkettige oder verzweigte $C_1$- bis $C_{20}$-Alkoxygruppen, vorzugsweise Methoxy, Ethoxy oder Ethylhexyloxy, und $R^8$ und $R^9$ gleich sind und stehen für H oder geradkettige oder verzweigte $C_1$- bis $C_{20}$-Alkoxygruppen, vorzugsweise Methoxy, Ethoxy oder Ethylhexyloxy, erfindungsgemäß besonders bevorzugt. Insbesondere ist es dabei bevorzugt, wenn und $R^8$ und $R^9$ für H stehen.

Die Verbindungen der Formel I werden erfindungsgemäß typisch in Mengen von 0,01 bis 20 Gew.-%, vorzugsweise in Mengen von 0,5 Gew.-% bis 10 Gew.-% und insbesondere bevorzugt in Mengen von 1 bis 8 Gew.-% eingesetzt. Dabei bereitet es dem Fachmann keinerlei Schwierigkeiten die Mengen abhängig von dem beabsichtigten Lichtschutzfaktor der Zubereitung entsprechend auszuwählen.

Erfindungsgemäß insbesondere bevorzugte Zubereitungen enthalten wie oben ausgeführt, neben den Verbindungen der Formel I weitere UV-Filter, vorzugsweise UV-B- und UV-A-I-Filter.

Bei Einsatz der als UV-A-Filter insbesondere bevorzugten Dibenzoylmethanderivate in Kombination mit den Verbindungen der Formel I ergibt sich ein zusätzlicher Vorteil: Die UV-empfindlichen Dibenzoylmethanderivate werden durch die Anwesenheit der Verbindungen der Formel I zusätzlich stabilisiert. Ein weiterer Gegenstand der vorliegenden Erfindung ist daher die Verwendung der Verbindungen gemäß Formel I zur Stabilisierung von Dibenzoylmethanderivaten in Zubereitungen.

Prinzipiell kommen alle UV-Filter für eine Kombination mit den erfindungsgemäßen Verbindungen der Formel I in Frage. Besonders bevorzugt sind solche UV-Filter, deren physiologische Unbedenklichkeit bereits nachgewiesen ist. Sowohl für UVA wie auch UVB-Filter gibt es viele aus der Fachliteratur bekannte und bewährte Substanzen, z.B.

Benzylidenkampferderivate wie 3-(4'-Methylbenzyliden)-dl-kampfer (z.B. Eusolex® 6300), 3-Benzylidenkampfer (z.B. Mexoryl® SD), Polymere von N-{(2 und 4)-[(2-oxoborn-3-yliden)methyl]benzyl}-acrylamid (z.B. Mexoryl® SW), N,N,N-Trimethyl-4-(2-oxoborn-3-ylidenmethyl)anilinium methylsulfat (z.B. Mexoryl® SK) oder (2-Oxoborn-3-yliden)toluol-4-sulfonsäure (z.B. Mexoryl® SL),

Benzoyl- oder Dibenzoylmethane wie 1-(4-tert-Butylphenyl)-3-(4-methoxyphenyl)propan-1,3-dion (z.B. Eusolex® 9020) oder 4-Isopropyldibenzoylmethan (z.B. Eusolex® 8020),

Benzophenone wie 2-Hydroxy-4-methoxybenzophenon (z.B. Eusolex® 4360) oder 2-Hydroxy-4-methoxybenzophenon-5-sulfonsäure und ihr Natriumsalz (z.B. Uvinul® MS-40),

Methoxyzimtsäureester wie Methoxyzimtsäureoctylester (z.B. Eusolex® 2292), 4-Methoxyzimtsäureisopentylester, z.B. als Gemisch der Isomere (z.B. Neo Heliopan® E 1000),

Salicylatderivate wie 2-Ethylhexylsalicylat (z.B. Eusolex® OS), 4-Isopropylbenzylsalicylat (z.B. Megasol®) oder 3,3,5-Trimethylcyclohexylsalicylat (z.B. Eusolex® HMS),

4-Aminobenzoesäure und Derivate wie 4-Aminobenzoesäure, 4-(Dimethylamino)benzoesäure-2-ethylhexylester (z.B. Eusolex® 6007), ethoxylierter 4-Aminobenzoesäureethylester (z.B. Uvinul® P25),

Phenylbenzimidazolsulfonsäuren, wie 2-Phenylbenzimidazol-5-sulfonsäure sowie ihre Kalium-, Natrium- und Triethanolaminsalze (z.B. Eusolex® 232), 2,2-(1,4-Phenylen)-bisbenzimidazol-4,6-disulfonsäure bzw. deren Salze (z.B. Neoheliopan® AP) oder 2,2-(1,4-Phenylen)-bisbenzimidazol-6-sulfonsäure;

und weitere Substanzen wie

- 2-Cyano-3,3-diphenylacrylsäure-2-ethylhexylester (z.B. Eusolex® OCR),
- 3,3'-(1,4-Phenylendimethylen)-bis-(7,7-dimethyl-2-oxobicyclo-[2.2.1]hept-1-ylmethansulfonsäure sowie ihre Salze (z.B. Mexoryl® SX) und
- 2,4,6-Trianilino-(p-carbo-2'-ethylhexyl-1 '-oxi)-1,3,5-triazin ( z.B. Uvinul® T 150)
- 2-(4-Diethylamino-2-hydroxy-benzoyl)-benzoesäure hexylester (z.B. Uvinul®UVA Plus, Fa. BASF).

Die in der Liste aufgeführten Verbindungen sind nur als Beispiele aufzufassen. Selbstverständlich können auch andere UV-Filter verwendet werden.

Diese organischen UV-Filter werden in der Regel in einer Menge von 0,5 bis 10 Gewichtsprozent, vorzugsweise 1 - 8 %, in kosmetische Formulierungen eingearbeitet.

Weitere geeignete organische UV-Filter sind z.B.

- 2-(2H-Benzotriazol-2-yl)4-methyl-6-(2-methyl-3-(1,3,3,3-tetramethyl-1-(trimethylsilyloxy)disiloxanyl)propyl)phenol (z.B. Silatrizole®),
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB),
- α-(Trimethylsilyl)-ω-[trimethylsilyl)oxy]poly[oxy(dimethyl [und ca. 6% methyl[2-[p-[2,2-bis(ethoxycarbonyl]vinyl] phenoxy]-1-methylenethyl] und ca. 1,5 % methyl[3-[p-[2,2-bis(ethoxycarbonyl)vinyl)phenoxy)-propenyl) und 0,1 bis 0,4% (methylhydrogen]silylen]] ($n \approx 60$) (CAS-Nr. 207 574-74-1)
- 2,2'-Methylen-bis-(6-(2H-benzotriazol-2-yl)-4-(1,1,3,3-tetramethylbutyl)phenol) (CAS-Nr. 103 597-45-1)
- 2,2'-(1,4-Phenylen)bis-(1 H-benzimidazol-4,6-disulfonsäure, Mononatriumsalz) (CAS-Nr. 180 898-37-7) und
- 2,4-bis-{[4-(2-Ethyl-hexyloxy)-2-hydroxyl]-phenyl}-6-(4-methoxyphenyl)-1,3,5-triazin (CAS-Nr. 103 597-45-, 187 393-00-6).
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB),

Organische UV-Filter werden in der Regel in einer Menge von 0,5 bis 20 Gewichtsprozent, vorzugsweise 1 - 15 %, in kosmetische Formulie-rungen eingearbeitet.

Als anorganische UV-Filter sind solche aus der Gruppe der Titandioxide wie z.B. gecoatetes Titandioxid (z. B. Eusolex® T-2000, Eusolex® T-AQUA), Zinkoxide (z.B. Sachtotec®), Eisenoxide oder auch Ceroxide denkbar. Diese anorganischen UV-Filter werden in der Regel in einer Menge von 0,5 bis 20 Gewichtsprozent, vorzugsweise 2 - 10 %, in kosmetische Zubereitungen eingearbeitet.

Bevorzugte Verbindungen mit UV-filternden Eigenschaften sind 3-(4'-Methylbenzyliden)-dl-kampfer, 1-(4-tert-Butylphenyl)-3-(4-methoxy-phenyl)-pro-pan-1,3-dion, 4-Isopropyldibenzoylmethan, 2-Hydroxy-4-meth-oxy--ben-zo--phenon, Methoxyzimtsäureoctylester, 3,3,5-Trimethyl-cyclo-hexylsali-cylat, 4-(Dimethylamino)benzoesäure-2-ethylhexylester, 2-Cyano-3,3-di-phenyl-acrylsäure-2-ethylhexylester, 2-Phenyl-benzimidazol-5-sulfonsäure sowie ihre Kalium-, Natrium- und Triethanol-aminsalze.

Durch Kombination von einer oder mehrerer Verbindungen der Formel I mit weiteren UV-Filtern kann die Schutzwirkung gegen schädliche Einwirkungen der UV-Strahlung optimiert werden.

Optimierte Zusammensetzungen können beispielsweise die Kombination der organischen UV-Filter 4'-Methoxy-6-hydroxyflavon mit 1-(4-tert-Butylphenyl)-3-(4-methoxyphenyl)propan-1,3-dion und 3-(4'-Methylbenzyliden)-dl-kampfer enthalten. Mit dieser Kombination ergibt sich ein Breitbandschutz, der durch Zusatz von anorganischen UV-Filtern, wie Titandioxid-Mikropartikeln noch ergänzt werden kann.

Alle genannten UV-Filter sowie die Verbindungen der Formel I können auch in verkapselter Form eingesetzt werden. Insbesondere ist es von Vorteil organische UV-Filter in verkapselter Form einzusetzen. Im Einzelnen ergeben sich die folgende Vorteile:

- Die Hydrophilie der Kapselwand kann unabhängig von der Löslichkeit des UV-Filters eingestellt werden. So können beispielsweise auch hydrophobe UV-Filter in rein wässrige Zubereitungen eingearbeitet werden. Zudem wird der häufig als unangenehm empfundene ölige Eindruck beim Auftragen der hydrophobe UV-Filter enthaltenden Zubereitung unterbunden.
- Bestimmte UV-Filter, insbesondere Dibenzoylmethanderivate, zeigen in kosmetischen Zubereitungen nur eine verminderte Photostabilität. Durch Verkapselung dieser Filter oder von Verbindungen, die die Photostabilität dieser Filter beeinträchtigen, wie beispielsweise Zimtsäurederivate, kann die Photostabilität der gesamten Zubereitung erhöht werden.
- In der Literatur wird immer wieder die Hautpenetration durch organische UV-Filter und das damit verbundene Reizpotential beim direkten Auftragen auf die menschliche Haut diskutiert. Durch die hier vorgeschlagene Verkapselung der entsprechenden Substanzen wird dieser Effekt unterbunden.

- Allgemein können durch Verkapselung einzelner UV-Filter oder anderer Inhaltstoffe Zubereitungsprobleme, die durch Wechselwirkung einzelner Zubereitungsbestandteile untereinander entstehen, wie Kristallisationsvorgänge, Ausfällungen und Agglomeratbildung vermieden werden, da die Wechselwirkung unterbunden wird.

Daher ist es erfindungsgemäß bevorzugt, wenn ein oder mehrere der Verbindungen gemäß Formel I bzw. der oben genannten UV-Filter in verkapselter Form vorliegen. Vorteilhaft ist es dabei, wenn die Kapseln so klein sind, dass sie mit dem bloßen Auge nicht beobachtet werden können. Zur Erzielung der o.g. Effekte ist es weiterhin erforderlich, dass die Kapseln hinreichend stabil sind und den verkapselten Wirkstoff (UV-Filter) nicht oder nur in geringem Umfang an die Umgebung abgeben.

Geeignete Kapseln können Wände aus anorganischen oder organischen Polymeren aufweisen. Beispielsweise wird in US 6,242,099 B1 die Herstellung geeigneter Kapseln mit Wänden aus Chitin, Chitin-Derivaten oder polyhydroxylierten Polyaminen beschrieben. Erfindungsgemäß besonders bevorzugt einzusetzende Kapseln weisen Wände auf, die durch einen SolGel-Prozeß, wie er in den Anmeldungen WO 00/09652, WO 00/72806 und WO 00/71084 beschrieben ist, erhalten werden können. Bevorzugt sind hier wiederum Kapseln, deren Wände aus Kieselgel (Silica; undefiniertes Silicium-oxid-hydroxid) aufgebaut sind. Die Herstellung entsprechender Kapseln ist dem Fachmann beispielsweise aus den zitierten Patentanmeldungen bekannt, deren Inhalt ausdrücklich auch zum Gegenstand der vorliegenden Anmeldung gehört.

Dabei sind die Kapseln in erfindungsgemäßen Zubereitungen vorzugsweise in solchen Mengen enthalten, die gewährleisten, dass die verkapselten UV-Filter in den oben angegebenen Mengen in der Zubereitung vorliegen.

Weisen die erfindungsgemäß einzusetzenden Verbindungen freie Hydroxy-Gruppen auf, so zeigen sie neben den beschriebenen Eigenschaften zusätzlich eine Wirkung als Antioxidans und/oder Radikalfänger. Bevorzugt sind daher auch Zubereitungen mit Lichtschutzeigenschaften enthaltend zumindest eine Verbindung der Formel I, die dadurch gekennzeichnet ist, dass mindestens einer der Reste $R^1$ bis $R^3$ steht für OH, wobei vorzugsweise mindestens einer der Reste $R^1$ oder $R^2$ für OH steht.

Damit die Verbindungen der Formel I ihre positive Wirkung als Radikalfänger auf die Haut besonders gut entwickeln können, kann es bevorzugt sein die Verbindungen der Formel I in tiefere Hautschichten eindringen zu lassen. Dazu stehen mehrere Möglichkeiten zur Verfügung. Zum einen können die Verbindungen der Formel I eine ausreichende Lipophilie aufweisen, um durch die äußere Hautschicht in epidermale Schichten vordringen zu können. Als weitere Möglichkeit können in der Zubereitung auch entsprechende Transportmittel, beispielsweise Liposomen, vorgesehen sein, die einen Transport der Verbindungen der Formel I durch die äußeren Hautschichten ermöglichen. Schließlich ist auch ein systemischer Transport der Verbindungen der Formel I denkbar. Die Zubereitung wird dann beispielsweise so gestaltet, dass sie für eine orale Gabe geeignet ist.

Allgemein wirken die Substanzen der Formel I als Radikalfänger. Solche Radikale werden nicht nur durch Sonnenlicht erzeugt, sondern werden unter verschiedenen Bedingungen gebildet. Beispiele sind Anoxie, die den Elektronenfluß stromauf der Cytochromoxidasen blockiert und die Bildung von Superoxidradikalarionen bedingt; Entzündungen, die unter anderem mit der Bildung von Superoxidanionen durch die Membran-NADPH-Oxidase der Leukozyten einhergehen, die jedoch auch mit der Bildung (durch Disproportionierung in Gegenwart von Eisen (II)-ionen) der Hydroxyradikale und anderer reaktiver Spezies, die normalerweise beim Phänomen einer Phagocytose beteiligt sind, einhergehen; sowie Lipidautooxidation die im Allgemeinen durch ein Hydroxylradikal initiiert wird und lipidische Alkoxyradikale und Hydroperoxide liefert.

Es wird vermutet, dass bevorzugte Verbindungen der Formel I auch als Enzymhemmer wirken. Sie hemmen vermutlich Histidindecarboxylase, Proteinkinasen, Elastase, Aldosereduktase sowie Hyaluronidase, und ermöglichen daher, die Unversehrtheit der Grundsubstanz vaskulärer Hüllen aufrecht zu erhalten. Ferner hemmen sie vermutlich nicht spezifisch Katechol-O-methyltransferase, wodurch die Menge der verfügbaren Katecholamine und dadurch die Gefäßfestigkeit erhöht wird. Weiter hemmen sie AMP-Phosphodiesterase, wodurch die Substanzen ein Potential zur Hemmung der Thrombozytenaggregation aufweisen.

Aufgrund dieser Eigenschaften eignen sich die erfindungsgemäßen Zubereitungen allgemein zur Immunprotektion und zum Schutz der DNA und RNA. Insbesondere eignen sich die Zubereitungen dabei zum Schutz von DNA und RNA vor oxidativen Angriffen, vor Radikalen und vor Schädigung durch Strahlung, insbesondere UV-Strahlung. Ein weiterer Vorteil der erfindungsgemäßen Zubereitungen ist der Zellschutz, insbesondere der Schutz von Langerhans-Zellen vor Schäden durch die oben genannten Einflüsse. Alle diese Verwendungen bzw. die Verwendung der Verbindungen der Formel I zur Herstellung entsprechend einsetzbarer Zubereitungen sind ausdrücklich auch Gegenstand der vorliegenden Erfindung.

Insbesondere eignen sich bevorzugte erfindungsgemäße Zusammensetzungen auch zur Behandlung von Hautkrankheiten, die mit einer Störung der Keratinisierung verbunden sind, die die Differenzierung und Zellprolif-eration betrifft, insbe-sondere zur Behandlung der Akne vulgaris, Akne comedonicá, der polymorphen Akne, der Akne rosaceae, der nodulären Akne, der Akne conglobata, der alters-bedingten Aknen, der als Neben-wirkung auftretenden Aknen, wie der Akne solaris, der medikamenten-bedingten Akne oder der Akne professionalis, zur Behandlung anderer

Störungen der Keratinisierung, insbesondere der Ichtyosen, der ichtyosi-formen Zustände, der Darrier-Krankheit, der Keratosis palmoplantaris, der Leukoplasien, der leukoplasiformen Zustände, der Haut- und Schleimhaut-flechten (Buccal) (Lichen), zur Behandlung anderer Hauterkrankungen, die mit einer Störung der Keratinisierung zusammenhängen und eine entzünd-liche und/oder immunoallergische Komponente haben und insbesondere aller Formen der Psoriasis, die die Haut, die Schleimhäute und die Finger und Zehennägel betreffen, und des psoriatischen Rheumas und der Hautatopien, wie Ekzemen oder der respiratorischen Atopie oder auch der Hypertrophie des Zahnfleisches, wobei die Verbindungen ferner bei einigen Entzündungen verwendet werden können, die nicht mit einer Störung der Keratinisierung zusammenhängen, zur Behandlung aller gutartigen oder bösartigen Wucherungen der Dermis oder Epidermis, die gegebenenfalls viralen Ursprungs sind, wie Verruca vulgaris. Veruca plana, Epidermodysplasia verruciformis, orale Papillomatose, Papillomatosis florida, und der Wucherungen, die durch UV-Strahlung hervorgerufen werden können, insbesondere des Epithelioma baso-cellulare und Epithelioma spinocellulare, zur Behandlung anderer Hautkrankheiten, wie der Dermatitis bullosa und der das Kollagen betreffenden Krankheiten, zur Behandlung bestimmter Augenkrankheiten, insbesondere der Hornhauterkran-kungen, zur Behebung oder Bekämpfung der lichtbedingten und der mit dem Älterwerden zusammenhängenden Hautalterung, zur Verminderung der Pigmentierungen und der Keratosis actinica und zur Behandlung aller Krankheiten, die mit der normalen Alterung oder der lichtbedingten Alterung zusammenhängen, zur Vorbeugung vor oder der Heilung von Wunden/Narben der Atrophien der Epidermis und/oder Dermis, die durch lokal oder systemisch angewendete Corticosteroide hervorgerufen werden und aller sonstigen Arten der Hautatrophie, zur Vorbeugung vor oder Behandlung von Störungen der Wundheilung, zur Vermeidung oder Behebung von Schwanger-schaftsstreifen oder auch zur Förderung der Wundheilung, zur Bekämpfung von Störungen der Talgproduktion, wie Hypersebhorrhö bei Akne oder der einfachen Seborrhö, zur Bekämpfung von oder Vorbeugung von krebsartigen Zuständen oder vor präkanzerogenen Zuständen, insbesondere der promyelozytären Leukämien, zur Behandlung von Entzündungserkrankungen, wie Arthritis, zur Behandlung aller virusbedingten Erkrankungen der Haut oder anderer Bereiche des Körpers, zur Vorbeugung vor oder Behandlung der Alopecie, zur Behandlung von Hautkrankheiten oder Krankheiten anderer Körperbereiche mit einer immuno-logischen Komponente, zur Behandlung von Herz-/Kreislauf-Erkran-kungen, wie Arteriosklerose oder Bluthochdruck, sowie des Insulin-unabhängigen Diabetes, zur Behandlung von Hautproblemen, die durch UV-Strahlung hervorgerufen werden.

Die schützende Wirkung gegen oxidativen Stress bzw. gegen die Einwirkung von Radikalen kann weiter verbessert werden, wenn die Zubereitungen ein oder mehrere weitere Antioxidantien enthalten.

In einer bevorzugten Ausführungsform der vorliegenden Erfindungen handelt es sich bei der Zubereitung daher um eine Zubereitung zum Schutz von Körperzellen gegen oxidativen Stress, insbesondere zur Verringerung der Hautalterung, dadurch gekennzeichnet, dass sie vorzugsweise ein oder mehrere Antioxidantien enthält.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, $ZnSO_4$), Selen und dessen Derivate (z.B. Selenmethionin), Stilbene und deren Derivate (z.B. Stilbenoxid, trans-Stilbenoxid).

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. (z.B. Oxynex® AP), natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L-(+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004). Derartige Antioxidantien werden mit Verbindungen der Formel I in solchen Zusammensetzungen überlicherweise in Verhältnissen im Bereich von 1000:1 bis 1:1000, bevorzugt

in Mengen von 100:1 bis 1:100 eingesetzt.

Die erfindungsgemäßen Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin $B_1$), Riboflavin (Vitamin $B_2$), Nicotinsäureamid, Vitamin C (Ascorbinsäure), Vitamin D, Ergocalciferol (Vitamin $D_2$), Vitamin E, DL-α-Tocopherol, Tocopherol-E-Acetat, Tocopherolhydrogensuccinat, Vitamin $K_1$, Esculin (Vitamin P-Wirkstoff), Thiamin (Vitamin $B_1$), Nicotinsäure (Niacin), Pyridoxin, Pyridoxal, Pyridoxamin, (Vitamin $B_6$), Panthothensäure, Biotin, Folsäure und Cobalamin (Vitamin $B_{12}$) in den erfindungsgemäßen kosmetischen Zubereitungen enthalten, insbesondere bevorzugt Vitamin-A-Palmitat, Vitamin C, DL-α-Tocopherol, Tocopherol-E-Acetat, Nicotinsäure, Pantothensäure und Biotin. Vitamine werden dabei mit Verbindungen der Formel I überlicherweise in Verhältnissen im Bereich von 1000:1 bis 1:1000, bevorzugt in Mengen von 100:1 bis 1:100 eingesetzt.

Die erfindungsgemäßen Zubereitungen können darüber hinaus weitere übliche hautschonende oder hautpflegende Wirkstoffe enthalten. Dies können prinzipiell alle den Fachmann bekannten Wirkstoffe sein.

Besonders bevorzugte Wirkstoffe sind Pyrimidincarbonsäuren und/oder Aryloxime.

Pyrimidincarbonsäuren kommen in halophilen Mikroorganismen vor und spielen bei der Osmoregulation dieser Organismen eine Rolle (*E. A. Galinski et al., Eur. J. Biochem., 149 (1985) Seite 135-139*). Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure und deren Derivate zu nennen. Diese Verbindungen stabilisieren Enzyme und andere Biomoleküle in wässrigen Lösungen und organischen Lösungsmitteln. Weiter stabilisieren sie insbesondere Enzyme gegen denaturierende Bedingungen, wie Salze, extreme pH-Werte, Tenside, Harnstoff, Guanidiniumchlorid und andere Verbindungen.

Ectoin und Ectoin-Derivate wie Hydroxyectoin können vorteilhaft in Arzneimitteln verwendet werden. Insbesondere kann Hydroxyectoin zur Herstellung eines Arzneimittels zur Behandlung von Hauterkrankungen eingesetzt werden. Andere Einsatzgebiete des Hydroxyectoins und anderer Ectoin-Derivate liegen typischerweise in Gebieten in denen z.B. Trehalose als Zusatzstoff verwendet wird. So können Ectoin-Derivate, wie Hydroxyectoin, als Schutzstoff in getrockneten Hefe- und Bakterienzellen Verwendung finden. Auch pharmazeutische Produkte wie nicht glykosylierte, pharmazeutische wirksame Peptide und Proteine z.B. t-PA können mit Ectoin oder seinen Derivaten geschützt werden.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP-A-0 671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Make-Ups, Pflegecremes und Sonnenschutzpräparaten eingesetzt werden.

Dabei wird vorzugsweise eine Pyrimidincarbonsäure gemäß der unten stehenden Formel II eingesetzt,

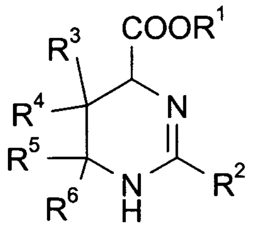

II

worin $R^1$ ein Rest H oder C1-8-Alkyl, $R^2$ ein Rest H oder C14-Alkyl und $R^3$, $R^4$, $R^5$ sowie $R^6$ jeweils unabhängig voneinander ein Rest aus der Gruppe H, OH, $NH_2$ und C1-4-Alkyl sind. Bevorzugt werden Pyrimidincarbonsäuren eingesetzt, bei denen $R^2$ eine Methyl- oder eine Ethylgruppe ist und $R^1$ bzw. $R^5$ und $R^6$ H sind. Insbesondere bevorzugt werden die Pyrimidincarbonsäuren Ectoin ((S)-1,4,5,6-Tetrahydro-2-methyl-4-pyrimidin-carbonsäure) und Hydroxyectoin ((S, S)-1,4,5,6-Tetrahydro-5-hydroxy-2-methyl-4-pyrimidin-carbonsäure) eingesetzt. Dabei enthalten die erfindungsgemäßen Zubereitungen derartige Pyrimidincarbonsäuren vorzugsweise in Mengen bis zu 15 Gew.-%. Vorzugsweise werden die Pyrimidincarbonsäuren dabei in Verhältnissen von 100:1 bis 1:100 zu den Verbindungen der Formel I eingesetzt, wobei Verhältnisse im Bereich 1:10 bis 10:1 besonders bevorzugt sind.

Unter den Aryloximen wird vorzugsweise 2-Hydroxy-5-methyllaurophenonoxim, welches auch als HMLO, LPO oder F5 bezeichnet wird, eingesetzt. Seine Eignung zum Einsatz in kosmetischen Mitteln ist beispielsweise aus der Deutschen Offenlegungsschrift DE-A-41 16 123 bekannt. Zubereitungen, die 2-Hydroxy-5-methyllaurophenonoxim enthalten, sind demnach zur Behandlung von Hauterkrankungen, die mit Entzündungen einhergehen, geeignet. Es ist bekannt, dass derartige Zubereitungen z.B. zur Therapie der Psioriasis, unterschiedlicher Ekzemformen, irritativer und toxischer Dermatitis, UV-Dermatitis sowie weiterer allergischer und/oder entzündlicher Erkrankungen der Haut und

der Hautanhangsgebilde verwendet werden können. Erfindungsgemäße Zubereitungen, die neben der Verbindung der Formel I zusätzlich eine Aryloxim, vorzugsweise 2-Hydroxy-5-methyllaurophenonoxim enthalten, zeigen überraschende antiinflammatorische Eignung. Dabei enthalten die Zubereitungen vorzugsweise 0,01 bis 10 Gew.-% des Aryloxims, wobei es insbesondere bevorzugt ist, wenn die Zubereitung 0,05 bis 5 Gew-% Aryloxim enthält.

Alle Verbindungen oder Komponenten, die in den Zubereitungen verwendet werden können, sind entweder bekannt und käuflich erwerbbar oder können nach bekannten Verfahren synthetisiert werden.

Die eine oder die mehreren Verbindungen der Formel I können in der üblichen Weise in kosmetische Zubereitungen eingearbeitet werden. Geeignet sind Zubereitungen für eine äußerliche Anwendung, beispielsweise als Creme, Lotion, Gel, oder als Lösung, die auf die Haut aufgesprüht werden kann. Für eine innerliche Anwendung sind Darreichungsformeln wie Kapseln, Dragees, Pulver, Tabletten-Lösungen oder Lösungen geeignet.

Als Anwendungsform der erfindungsgemäßen Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays. Weitere Anwendungsformen sind z.B. Sticks, Shampoos und Duschbäder. Der Zubereitung können beliebige übliche Trägerstoffe, Hilfsstoffe und gegebenenfalls weitere Wirkstoffe zugesetzt werden.

Vorzuziehende Hilfsstoffe stammen aus der Gruppe der Konservierungsstoffe, Antioxidantien, Stabilisatoren, Lösungsvermittler, Vitamine, Färbemittel, Geruchsverbesserer.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

Zu den bevorzugten erfindungsgemäßen Zubereitungsformen gehören insbesondere Emulsionen.

Erfindungsgemäße Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:

- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettlkoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäure und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexaldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12}$-$_{15}$-Alkylbenzoat, Capryl-Caprinsäure-triglycerid, Dicaprylether.

Besonders vorteilhaft sind Mischungen aus $C_{12}$-$_{15}$-Alkylbenzoat und 2-Ethylhexylisostearat, Mischungen aus $C_{12}$-$_{15}$-Alkylbenzoat und Isotridecylisononanoat sowie Mischungen aus $C_{12}$-$_{15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann auch die Ölphase ferner einen Gehalt an cyclischen oder linearan Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemisch der vorstehend genannten Lösemittel verwendet. Bei alkoholischen Lösemitteln kann Wasser ein weiterer Bestandteil sein.

Erfindungsgemäße Emulsionen sind vorteilhaft und enthalten z. B. die genannten Fette, Öle, Wachse und anderen Fettkörper, sowie Wasser und einen Emulgator, wie er üblicherweise für einen solchen Typ der Formuierung verwendet wird.

In einer bevorzugten Ausführungsform enthalten die erfindungsgemäßen Zubereitungen hydrophile Tenside.

Die hydrophilen Tenside werden bevorzugt gewählt aus der Gruppe der Alkylglucoside, der Acyllactylate, der Betaine sowie der Cocoamphoacetate.

Die Alkylglucoside werden ihrerseits vorteilhaft gewählt aus der Gruppe der Alkylglucoside, welche sich durch die Strukturformel

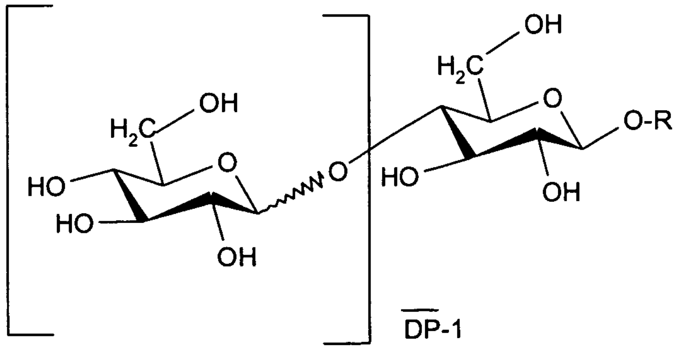

auszeichnen, wobei R einen verzweigten oder unverzweigten Alkylrest mit 4 bis 24 Kohlenstoffatomen darstellt und wobei $\overline{DP}$ einen mittleren Glucosylierungsgrad von bis zu 2 bedeutet.

Der Wert $\overline{DP}$ repräsentiert den Glucosidierungsgrad der erfindungsgemäß verwendeten Alkylglucoside und ist definiert als

$$\overline{DP} = \frac{p_1}{100} \cdot 1 + \frac{p_2}{100} \cdot 2 + \frac{p_3}{100} \cdot 3 + ... = \sum \frac{p_i}{100} \cdot i$$

Dabei stellen $p_1$, $p_2$, $p_3$ ... bzw. $p_i$ den Anteil der einfach, zweifach dreifach ... i-fach glucosylierten Produkte in Gewichtsprozenten dar. Erfindungsgemäß vorteilhaft werden Produkte mit Glucosylierungsgraden von 1-2, insbesondere vorteilhaft von 1, 1 bis 1,5, ganz besonders vorteilhaft von 1,2-1,4, insbesondere von 1,3 gewählt.

Der Wert DP trägt den Umstande Rechnung, dass Alkylglucoside herstellungsedingt in der Regel Gemische aus Mono- und Oligoglucosiden darstellen. Erfindungsgemäß vorteilhaft ist ein relativ hoher Gehalt an Monoglucosiden, typischerweise in der Größenordnung von 40-70 Gew.-%.

Erfindungsgemäß besonders vorteilhaft verwendete Alkylglylcoside werden gewählt aus der Gruppe Octylglucopyranosid, Nonylglucopyranosid, Decylglucopyranosid, Undecylglucopyranosid, Dodecylglucopyranosid, Tetradecylglucopyranosid und Hexadecylglucopyranosid.

Es ist ebenfalls von Vorteil, natürliche oder synthetische Roh- und Hilfsstoffe bzw. Gemische einzusetzen, welche sich durch einen wirksamen Gehalt an den erfindungsgemäß verwendeten Wirkstoffen auszeichnen, beispielsweise Plantaren® 1200 (Henkel KGaA), Oramix® NS 10 (Seppic).

Die Acyllactylate werden ihrerseits vorteilhaft gewählt aus der Gruppe der Substanzen, welche sich durch die Strukturformel

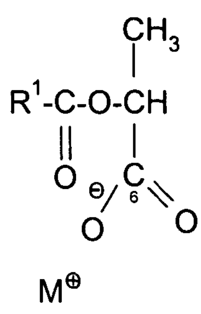

auszeichnen, wobei $R^1$ einen verzweigten oder unverzweigten Alkylrest mit 1 bis 30 Kohlenstoffatomen bedeutet und $M^+$ aus der Gruppe der Alkaliionen sowie der Gruppe der mit einer oder mehreren Alkyl- und/oder mit einer oder mehreren Hydroxyalkylresten substituierten Ammoniumionen gewählt wird bzw. dem halben Äquivalent eines Erdalkalions entspricht.

Vorteilhaft ist beispielsweise Natriumisostearyllactylat, beispielsweise das Produkt Pathionic® ISL von der Gesellschaft American Ingredients Company.

Die Betaine werden vorteilhaft gewählt aus der Gruppe der Substanzen, welche sich durch die Strukturformel

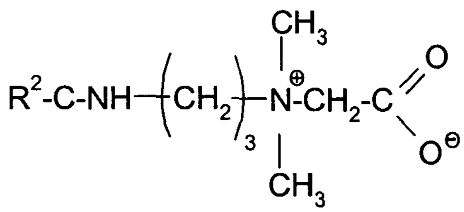

auszeichnen, wobei $R^2$ einen verzweigten oder unverzeigten Alkylrest mit 1 bis 30 Kohlenstoffatomen bedeutet.

Insbesondere vorteilhaft bedeutet $R^2$ einen verzweigten oder unverzweigten Alkylrest mit 6 bis 12 Kohlenstoffatomen.

Vorteilhaft ist beispielsweise Capramidopropylbetain, beispielsweise das Produkt Tego® Betain 810 von der Gesellschaft Th. Goldschmidt AG.

Als erfindungsgemäß vorteilhaftes Cocoamphoacetat wird beispielsweise Natriumcocoamphoacetat gewählt, wie es unter der Bezeichnung Miranol® Ultra C32 von der Gesellschaft Miranol Chemical Corp. erhältlich ist.

Die erfindungsgemäßen Zubereitungen sind vorteilhaft dadurch gekennzeichnet, dass das oder die hydrophilen Tenside in Konzentrationen von 0,01-20 Gew.-% bevorzugt 0,05-10 Gew.-%, besonders bevorzugt 0,1-5 Gew.-%, jeweils bezogen auf das Gesamtgewicht der Zusammensetzung, vorliegt oder vorliegen.

Zu Anwendung werden die erfindungsgemäßen kosmetischen und dermatologischen Zubereitungen in der für Kosmetika üblichen Weise aufdie Haut und/oder die Haare in ausreichender Menge aufgebracht.

Erfindungsgemäße kosmetische und dermatologische Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Waser-in-Öl-in-Wasser (W/O/W), ein Gel, einen festen Stift, eine Salbe oder auch ein Aerosol darstellen. Es ist auch vorteilhaft, Ectoine in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt. Bevorzugt werden Emulsionen. O/W-Emulsinen werden besonders bevorzugt. Emulsionen, W/O-Emulsionen und O/W-Emulsionen sind in üblicher Weise erhältlich.

Als Emulgatoren können beispielsweise die bekannten W/O- und O/W-Emulgatoren verwendet werden. Es ist vorteilhaft, weitere übliche Coemulgatoren in den erfindungsgemäßen bevorzugten O/W-Emulsionen zu verwenden.

Erfindungsgemäß vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen. Insbesondere bevorzugt sind: Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)-stearylether (Steareth-17),Polyethylenglycol(18) stearylether (Steareth-18), Polyethylenglycol(19)stearylether (Steareth-19), Polyethylenglycol(20)-stearylether (Steareth-20), Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)-isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol(20)isostearylether (Isosteareth-20), Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)-cetylether (Ceteth-18), Polyethylenglycol(19)cetylether (Ceteth-19), Polyethylen-glycol(20)cetylether (Ceteth-20), Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16) isocetylether (Isoceteth-16), Polyethylenglycol(17)-isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20), Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15), Polyethylenglycol(12)laurylether (Laureth-12), Polyethy-

lenglycol(12)-isolaurylether (Isolaureth-12), Polyethylenglycol(13)cetylstearylether (Ceteareth-13), Polyethylenglycol (14)cetylstearylether (Ceteareth-14), Polyethylenglycol(15)cetylstearylether (Ceteareth-15), Polyethylenglycol(16)cetylstearylether (Ceteareth-16), Polyethylenglycol(17)-cetylstearylether (Ceteareth-17), Polyethylenglycol(18)cetylstearylether (Ceteareth-18), Polyethylenglycol(19)cetylstearylether (Ceteareth-19), Polyethylenglycol(20)cetylstearylether (Ceteareth-20).

Es ist ferner von Vorteil, die Fettsäureethoxylate ausfolgender Gruppe zu wählen:

Polyethylenglycol(20)stearat, Polyethylenglycol(21 )stearat,
Polyethylenglycol(22)stearat, Polyethylenglycol(23)stearat,
Polyethylenglycol(24)stearat, Polyethylenglycol(25)stearat,
Polyethylenglycol(12)isostearat, Polyethylenglycol(13)isostearat,
Polyethylenglycol(14)isostearat, Polyethylenglycol(15)isostearat,
Polyethylenglycol(16)isostearat, Polyethylenglycol(17)isostearat,
Polyethylenglycol(18)isostearat, Polyethylenglycol(19)isostearat,
Polyethylenglycol(20)isostearat, Polyethylenglycol(21)isostearat,
Polyethylenglycol(22)isostearat, Polyethylenglycol(23)isostearat,
Polyethylenglycol(24)isostearat, Polyethylenglycol(25)isostearat,
Polyethylenglycol(12)oleat, Polyethylenglycol(13)oleat,
Polyethylenglycol(14)oleat, Polyethylenglycol(15)oleat,
Polyethylenglycol(16)oleat, Polyethylenglycol(17)oleat,
Polyethylenglycol(18)oleat, Polyethylenglycol(19)oleat,
Polyethylenglycol(20)oleat,

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol (25)Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20) glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/cprinat, Polyethylenglycol(20)glyceryloleat, Polyethylenglycol(20)glycerylisostearat, Polyethylenglycol(18)glyceryloleat(cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als fakultative, dennoch erfindungsgemäß gegebenenfalls vorteilhafte W/O-Emulgatoren können eingesetzt werden:

Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C-Atome, Diglycerinester gesättigter und/oder ungesättigter, verzweigter und/oder unverzweigter Alkancarbonsäuren einer Kettenlänge von 8 bis 24, insbesondere 12-18 C-Atomen, Monoglycerinether gesättigter und/oder ungesättigter, verzweigter und/ oder unverzweigter Alkohole einer Kettenlänge von 8 bis 24, insbesondere 12-18 C-Atomen, Diglycerinether gesättigter und/oder ungesättigter, verzweigter und/oder unverzweigter Alkhole einer Kettenlänge von 8 bis 24, insbesondere 12-18 C-Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Erfindungsgemäß bevorzugte Zubereitungen eignen sich besonders zum Schutz menschlicher Haut gegen Alterungsprozesse sowie vor oxidativem Stress, d.h. gegen Schädigungen durch Radikale, wie sie z.B. durch Sonneneinstrahlung, Wärme oder andere Einflüsse erzeugt werden. Dabei liegt sie in verschiedenen, für diese Anwendung üblicherweise verwendeten Darreichungsformen vor. So kann sie insbesondere als Lotion oder Emulsion, wie als Creme oder Milch (O/W, W/O, O/W/O, W/O/W), in Form ölig-alkoholischer, ölig-wässriger oder wässrigalkoholischer

Gele bzw. Lösungen, als feste Stifte vorliegen oder als Aerosol konfektioniert sein.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -milch vorliegt und außer der oder den Verbindungen der Formel I beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die erfindungsgemäße Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane.

Die kosmetische Zubereitung kann auch zum Schutz der Haare gegen fotochemische Schäden verwendet werden, um Veränderungen von Farbnuancen, ein Entfärben oder Schäden mechanischer Art zu verhindern. In diesem Fall erfolgt geeignet eine Konfektionierung als Shampoo, Lotion, Gel oder Emulsion zum Ausspülen, wobei die jeweilige Zubereitung vor oder nach dem Shamponieren, vor oder nach dem Färben oder Entfärben bzw. vor oder nach der Dauerwelle aufgetragen wird. Es kann auch eine Zubereitung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Die Zubereitung mit Lichtschutzeigenschaften kann außer der oder den Verbindungen der Formel I verschiedene, in diesem Mitteltyp verwendete Adjuvantien enthalten, wie Grenzflächen aktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Weitere Gegenstände der vorliegenden Erfindung sind ein Verfahren zur Herstellung einer Zubereitung, welches dadurch gekennzeichnet ist, dass mindestens eine Verbindung der Formel I mit Resten wie oben beschrieben mit einem kosmetisch oder dermatologisch geeigneten Träger vermischt wird, und die Verwendung einer Verbindung der Formel I zur Herstellung einer Zubereitung mit Lichtschutzeigenschaften.

Die erfindungsgemäßen Zubereitungen können dabei mit Hilfe von Techniken hergestellt werden, die dem Fachmann wohl bekannt sind.

Das Vermischen kann ein Lösen, Emulgieren oder Dispergieren der Verbindung gemäß Formel I in dem Träger zur Folge haben.

In einem erfindungsgemäß bevorzugten Verfahren wird die Verbindung nach Formel I hergestellt durch Umsetzung einer 2-Hydroxyacetophenon-Verbindung mit einer Lithiumverbindung und anschließend mit einer Ketoverbindung.

Beispielsweise können gemäß M. Cushman und D. Nagarathnam in: Tetrahedron Letters, 31, 6497-6500, 1990 und M.Cushman; D. Nagarathnam; Journal of Organic Chemistry", 56, 4884-4887, 1991 mit einem großen Überschuß an Lithiumbis(trimethylsilyl)amid unter homogenen Reaktions-bedingungen die phenolischen Hydroxylgruppen deprotoniert werden, um das Lithiumenolat des entsprechenden Ketons herzustellen. Anschließend kann das Kohlenstoffatom des Lithiumenolates regioselektiv mit einem Aroylchlorid acyliert werden, um so direkt ein β-Diketon Zwischenprodukt zu erhalten, welches anschließend im sauren Medium cyclisiert wird. Nachteilig an diesem Verfahren ist jedoch der große Überschuß an der Lithiumbase, der auch bei mehreren Reinigungsschritten nur schwer entfernbar ist, sowie der hohe Preis der Lithiumbase.

Daher ist es besonders bevorzugt ein Verfahren entsprechend der älteren Internationalen Patentanmeldung mit dem Anmelde-Aktenzeichen 02/00233 durchzuführen. Bei diesem erfindungsgemäß bevorztugten Verfahren wird das Verhältnis der molaren Äquivalente von Lithiumverbindung zu den zu metallierenden funktionellen Gruppen der 2-Hydroxyacetophenon-Verbindung im Bereich 1 bis 1,2 gewählt.

Überraschenderweise wurde gefunden, dass vorerwähntes Verhältnis eine vollständige Metallierung aller Hydroxylgruppen und der Carbonylgruppe der 2-Hydroxyacetophenon Verbindung erlaubt. Weniger als ein Verhältnis von 1 würde zu einer unvollständigen Metallierung führen und damit zu einer großen Anzahl an unerwünschten Nebenprodukten. Ein Verhältnis von mehr als 1,2 hingegen bedeutet den Einsatz einer größeren Menge der zumeist nicht preiswerten Lithiumverbindungen und das Mitschleppen von Lithiumverbindungen bei allen weiteren Folge- insbesondere Reinigungsschritten.

Bevorzugt ist die Lithiumverbindung ausgewählt aus anorganischen Lithiumverbindungen, da sie preiswert und einfach in großen Mengen verfügbar sind. Des weiteren bieten sie den Vorteil, dass sie in organischen Lösungsmitteln wenig bis gar nicht löslich sind, so dass sie nach einer unter heterogenen Bedingungen geführten Metallisierungsreaktion leicht aus der Reaktionsmischung filtriert werden können, wenn sie im Überschuß eingesetzt werden.

In einer bevorzugten Ausführungsform des erfindungsgemäßen Verfahrens beträgt das Verhältnis von Lithiumverbindung zu den zu metallierenden funktionellen Gruppen der 2-Hydroxyacetophenon-Verbindung genau 1 beträgt. Damit wird erreicht, dass keine evtl. noch gelösten Lithiumverbindungen als Verunreinigungen im Endprodukt auftreten, da diese meist auch nicht durch Reinigungsschritte, wie Umkristallisieren aus den Zwischen- und Endprodukten entfernt werden können.

Vorteilhafterweise wird die Metallierung in einem etherischen Lösungsmittel durchgeführt, da dies die Metallierungsreaktion durch seine Polarität durch Ausbildung von Li-solvaten unterstützt, wodurch die Basizität der Lithiumbase gesteigert wird.

Das im Rahmen des erfindungsgemäßen Verfahrens eingesetzte 2-Hydroxyacetophenon weist bevorzugt folgende Struktur auf:

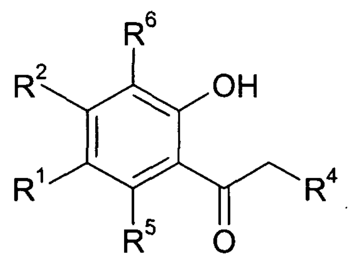

wobei $R^1$, $R^2$ und $R^4$ bis $R^6$ die oben angegebene Bedeutung haben oder Gruppen darstellen, die sich durch chemische Modifikationen, wie beispielsweise Abspaltung von Schutzgruppen, Oxidation oder Reduktion, in Gruppen mit den oben genannten Bedeutungen überführen lassen.

Die Ketoverbindung zur Durchführung des erfindungsgemäßen Verfahrens weist vorzugsweise folgende Struktur auf:

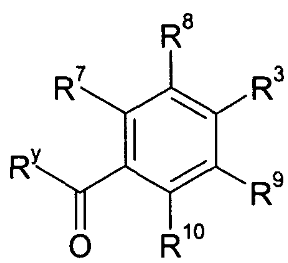

wobei $R^3$ und $R^7$ bis $R^{10}$ die oben angegebene Bedeutung haben oder Gruppen darstellen, die sich durch chemische Modifikationen, wie beispielsweise Abspaltung von Schutzgruppen, Oxidation oder Reduktion, in Gruppen mit den oben genannten Bedeutungen überführen lassen und wobei $R^y$ eine Halogenid-, Alkoxyl- oder Ester-Gruppe bedeuten kann.

Vorzugsweise sind die Hydroxylgruppen der 2-Hydroxyacetophenon Verbindung ungeschützt. Damit werden aufwendige Reaktionen zum Aufbringen und Entfernen von Schutzgruppen vermieden, so dass die Reaktion besonders einfach verläuft.

Im Falle der Ketoverbindungen bedeutet $R^y$ Chlorid, d.h. die Verbindung ist ein Säurechlorid, eine Alkoxylgruppe, d.h. die Verbindung ist ein Ester, oder eine Estergruppe, d.h. die Verbindung ist ein Säureanhydrid. Die Verwendung unterschiedlicher Gruppen erlaubt je nach eingesetztem Substrat auch die Variation und genaue Wahl der Reaktionszeit. Beispielsweise beträgt die Reaktionszeit bei Verwendung eines Säurechlorids oder eines Säureanhydrids zwischen 2-6 Stunden, meistens zwischen 4-5 Stunden. Bei Verwendnung eines Esters oder unter Verwendnung silylierter Schutzgruppen beträgt die Reaktionszeit mehr als 8 Stunden, zumeist mehr als 10, oftmals jedoch auch etwa 16-20 Stunden.

Zunächst wird die 2-Hydroxyacetophenon Verbindung vorzugsweise mit dem Keton und einer Lithiumverbindung in trockenem THF bei niedrigen Temperaturen kondensiert (-78°C bis -50 °C) und ergibt ein stabiles Diketon Zwischenprodukt. Bei Temperaturen von mehr als -50°C verläuft die Reaktion entweder gar nicht mehr oder zu schnell, d.h. mit unerwünschten Nebenprodukten oder aber unter Zersetzung des Ausgangsproduktes ab, so daß der Bereich von -78°C bis -50 °C bevorzugt ist. Anschließend wird das Diketon unter sauren Bedingungen bei 95-100°C cyclisiert, um ein Flavonderivat zu ergeben.

Als Lithiumbasen, die in einem erfindungsgemäßen Verfahren Verwendung finden, sind die nachstehend aufgeführten Lithiumbasen besonders geeignet:

$LiNH_2$, $LiN(CH_3)_2$, $LiN(C_2H_5)_2$,$LiNCH(CH_3)_2$ (LDA), $Me_3CLi$, $PhCH_2Li$, $Ph_2CHLi$, $Ph_3CLi$, LiCN, $LiC(NO_3)_3$, $LiC(CN)_3$, $LiN(C_6H_{11})_2$, $LiN(CH_2)_2$, $LiCH_3$, $LiC_2H_5$, $LiCH(CH_3)_2$, $LiC_4H_9$, $LiCH_2CH(CH_3)_2$, $LiC_6H_{13}$, LiPh, $LiCH_3COCHCOCH_3$, LiClO, $LiClO_4$, $LiIO_4$, $Li_2O$, LiOH, $LiOCH_3$, $LiOC_2H_5$, $LiOC_4H_9$, LiOPh, LiOOCOPh, Lithiumenolate der allgemeinen Formel $LiOCR=CR'_2$, wobei R und R' ein aliphatischer oder aromatischer Rest ist, $LiOSi(CH_3)_3$, $Li(Si(CH_3)_3)_2$, $Li_2CO_3$, Lithium-2,2,6,6-tetramethylpiperidin (LiTMP).

Wie vorstehend beschrieben, sind darunter die rein anorganischen Lithiumverbindungen bzw. diejenigen Lithiumverbindungen, deren zumeist organischer Rest über anorganische Atome (O, N, Si) an das Lithiumatom gebunden ist, besonders bevorzugt.

Als Lösungsmittel für die Durchführung der Metallisierungsreaktion wird wie vorstehend beschrieben bevorzugt ein etherisches Lösungsmittel, beispielsweise Diethylether, Tetrahydrofuran (THF), Dibutylether. Andere polare Lösungsmittel, wie Methylethylketon und dergleichen können jedoch ebenfalls verwendet werden, aber auch je nach eingesetztem Hydroxyacetophenon apolare Lösungsmittel, wie z.B. n-Hexan, Heptan, Benzol, Toluol usw.

Es wurde auch festgestellt, dass Verbindungen der Formel I stabilisierend auf die Zubereitung wirken können. Bei der Verwendung in entsprechenden Produkten bleiben diese daher auch länger stabil und verändern ihr Aussehen nicht. Insbesondere bleibt auch bei längerdauernder Anwendung bzw. längerer Lagerung die Wirksamkeit der Inhaltsstoffe, z.B. Vitamine, erhalten. Dies ist besonders vorteilhaft bei der Zusammensetzungen zum Schutz der Haut gegen die Einwirkung von UV-Strahlen, da diese Kosmetika besonders hohen Belastungen durch die UV-Strahlung ausgesetzt sind.

Die Antioxidanten Wirkungen der Verbindungen gemäß Formel I können beispielsweise mit dem 2,2-Diphenyl-1-picrylhydrazyl(DPPH)-Assay gezeigt werden. 2,2-Diphenyl-1-picrylhydrazyl ist ein in Lösung stabiles freies Radikal. Das ungepaarte Elektron führt zu einer starken Absorptionsbande bei 515 nm, die Lösung ist dunkel-violett gefärbt. In Gegenwart eines Radikalfängers wird das Elektron gepaart, die Absorption verschwindet und die Entfärbung verläuft stöchiometrisch unter Berücksichtigung der aufgenommenen Elektronen. Gemessen wird die Extinktion im Photometer. Die antiradikalische Eigenschaft der zu testenden Substanz wird bestimmt, indem man die Konzentration ermittelt, bei der 50 % des eingesetzten 2.2.Diphenyl-1-picrylhydrazyls mit dem Radikalfänger reagiert haben. Ausgedrückt wird diese Konzentration als $EC_{50}$, ein Wert, der unter den gegebenen Messbedingungen als Substanzeigenschaft zu betrachten ist. Verglichen wird die untersuchte Substanz mit einem Standard (z.B. Tocopherol)

Die Auswertung erfolgt grafisch, indem das molare Verhältnis Testsubstanz/DPPH gegen die prozentuale Extinktionsabnahme aufgetragen und die $EC_{50}$ durch Ablesen bei 50% ermittelt wird. Zusätzlich wird die Steigung der Graden im linearen Bereich ermittelt und der $EC_{50}$ errechnet.

Die positiven Wirkungen von Verbindungen der Formel I ergeben deren besondere Eignung zur Verwendung in kosmetischen oder pharmazeutischen Zubereitungen.

Ebenso positiv sind die Eigenschaften von Verbindungen mit der Formel I zu werten für eine Verwendung in Nahrungsmitteln oder als Nahrungsergänzungsmittel oder als "functional food". Die weiteren zu Nahrungsmitteln ausgeführten Erläuterungen gelten sinngemäß auch für Nahrungsergänzungsmittel und für "functional food".

Die Nahrungsmittel, die nach der vorliegenden Erfindung mit einer oder mehreren Verbindungen der Formel I angereichert werden können, umfassen alle Materialien, die für den Verzehr durch Tiere oder für den Verzehr durch Menschen geeignet sind, beispielsweise Vitamine und Provitamine davon, Fette, Mineralien oder Aminosäuren ". (Die Nahrungsmittel können fest sein aber auch flüssig, also als Getränk vorliegen). Nahrungsmittel, die nach der vorliegenden Erfindung mit einer oder mehreren Verbindungen der Formel I angereichert werden können, sind beispielsweise auch Nahrungsmittel, die aus einer einzigen natürlichen Quelle stammen, wie z.B. Zucker, ungesüßter Saft, Nektar oder Püree von einer einzigen Pflanzenspezies, wie z.B. ungesüßter Apfelsaft (z.B. auch eine Mischung verschiedener Sorten Apfelsaft), Grapefruitsaft, Orangensaft, Apfelkompott, Aprikosennektar, Tomatensaft, Tomatensoße, Tomatenpüree usw. Weitere Beispiele für Nahrungsmittel, die nach der vorliegenden Erfindung mit einer oder mehreren Verbindungen der Formel I angereichert werden können, sind Korn oder Getreide einer einzigen Pflanzenspezies und

Materialien, die aus derartigen Pflanzenspezies hergestellt werden, wie z.B. Getreidesirup, Roggenmehl, Weizenmehl oder Haferkleie. Auch Mischungen von derartigen Nahrungsmitteln sind geeignet, um nach der vorliegenden Erfindung mit einer oder mehreren Verbindungen der Formel I angereichert zu werden, beispielsweise Multivitaminpräparate, Mineralstoffmischungen oder gezuckerter Saft. Als weitere Beispiele für Nahrungsmittel, die nach der vorliegenden Erfindung mit einer oder mehreren Verbindungen der Formel I angereichert werden können, seien Nahrungsmittelzubereitungen, beispielsweise zubereitete Cerealien, Gebäck, Mischgetränke, speziell für Kinder zubereitete Nahrungsmittel, wie Joghurt, Diätnahrungsmittel, kalorienarme Nahrungsmittel oder Tierfutter, genannt.

Die Nahrungsmittel, die nach der vorliegenden Erfindung mit einer oder mehreren Verbindungen der Formel I angereichert werden können, umfassen somit alle genießbaren Kombinationen von Kohlehydraten, Lipiden, Proteinen, anorganischen Elementen, Spurenelementen, Vitaminen, Wasser oder aktiven Metaboliten von Pflanzen und Tieren.

Die Nahrungsmittel, die nach der vorliegenden Erfindung mit einer oder mehreren Verbindungen der Formel I angereichert werden können, werden vorzugsweise oral angewendet, z.B. in Form von Speisen, Pillen, Tabletten, Kapseln, Pulver, Sirup, Lösungen oder Suspensionen.

Die mit einer oder mehreren Verbindungen der Formel I angereicherten erfindungsgemäßen Nahrungsmittel können mit Hilfe von Techniken hergestellt werden, die dem Fachmann wohl bekannt sind.

Durch ihre Wirkung als Antioxidationsmittel bzw. als Radikalfänger eignen sich Verbindungen der Formel auch als Arzneimittelinhaltsstoff. Sie wirken dabei unterstützend oder substituierend zu natürlichen Mechanismen, welche Radikale im Körper abfangen. Die Verbindungen der Formel I können in ihrer Wirkung teilweise mit Radikalfängern wie Vitamin C verglichen werden. Verbindungen der Formel I können beispielsweise zur vorbeugenden Behandlungen von Entzündungen und Allergien der Haut sowie in bestimmten Fällen zur Verhütung bestimmter Krebsarten verwendet werden. Insbesondere eignen sich Verbindungen der Formel I zur Herstellung eines Arzneimittels zur Behandlung von Entzündungen, Allergien und Irritationen, insbesondere der Haut. Ferner können Arzneimittel hergestellt werden in einer Wirkung als Venentonikum, als Mittel zur Erhöhung der Festigkeit von Blutkapillaren, als Hemmstoff für Cuperose, als Hemmstoff chemischer, physikalischer oder aktinischer Erytheme, als Mittel zur Behandlung empfindlicher Haut, als Dekongestionsmittel, als Entwässerungsmittel, als Mittel zum Schlankmachen, als Antifaltenmittel, als Stimulatoren der Synthese von Komponenten der extrazellulären Matrix, als stärkendes Mittel zur Verbesserung der Hautelastizität und als Antialterungsmittel. Weiter zeigen in diesem Zusammenhang bevorzugte Verbindungen der Formel I antiallergische und antiinflammatorische und antiirritative Wirkungen. Sie eignen sich daher zur Herstellung von Arzneimitteln zur Behandlung von Entzündungen oder allergischen Reaktionen.

Ein weiterer Gegenstand der Erfindung betrifft; wie bereits oben erwähnt, die Stabilisierung von UV-Filtern. Eine bekannte und leistungsfähige Klasse von Lichtschutzfiltersubstanzen wird von den Dibenzoylmethanderivaten gebildet. Nachteilig ist jedoch, dass diese Substanzen sehr leicht durch UV-Licht zersetzt werden und damit ihre schützenden Eigenschaften verlorengehen. Als Beispiel für einen auf dem Markt erhältlichen Lichtschutzfilter aus dieser Verbindungsklasse sei das 4-(tert.-Butyl)-4'-methoxydibenzoylmethan angeführt, welches die unten gezeigte Struktur aufweist.

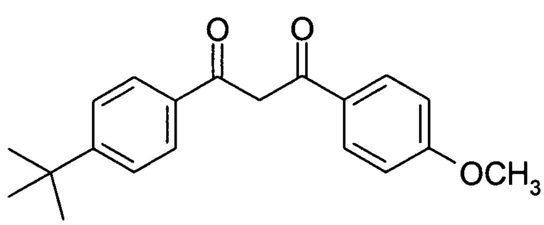

Überraschend hat sich nun gezeigt, dass Verbindungen der Formel I eine Stabilisierungswirkung für die Dibenzoylmethane, insbesondere 4-(tert.butyl)-4-methoxibenzoylmethan, aufweisen. Indem Gemische dieser Verbindungen in Kosmetika eingearbeitet werden, ist es nun möglich, Lichtschutzmittel unter Verwendung von Dibenzoylmethanen herzustellen, die auch bei längerer Sonneneinwirkung, beispielsweise während eines mehrstündigen Sonnenbades, keine oder nur eine geringe Verringerung der Schutzwirkung gegen UV-Strahlen aufweisen.

Im folgenden wird die Erfindung anhand von Beispielen näher erläutert.

**Beispiele**

| Chemikalien | Herkunft | Art.Nr. | Reinheit |
|---|---|---|---|
| 2',5'-Dihydroxyacetophenone | Merck KGaA | | 98% |
| 2',4'-Dihydroxyacetophenone | Merck KGaA | 818284 | 98% |
| 4-Methoxybensoesäurechlorid | Merck KGaA | 820106 | 99% |
| Lithiumhydroxid | Merck KGaA | 105691 | 98% |
| D-(+)-alpha-acetobromglucose | Merck KGaA | 800121 | |
| Tetra-n-butylammoniumbromid | Merck KGaA | 818839 | |
| Natronlauge | Merck KGaA | 109137 | 1N |
| Bortribromid | Merck KGaA | 801063 | 99% |
| NatriumMethylat | Merck KGaA | 806538 | |
| Tetrahydrofuran | Merck KGaA | 108107 | SeccoSolv 0,0075% $H_2O$ |
| Dichloromethan | Merck KGaA | 106049 | reinst |
| Salzsäure rauchend | Merck KGaA | 100314 | 37% |
| Essigsäure | Merck KGaA | 100056 | 100% |
| Schwefelsäure | Merck KGaA | 100731 | 95-97% |

**Beispiel 1 Herstellung von 4'-Methoxy-6-hydroxyflavon**

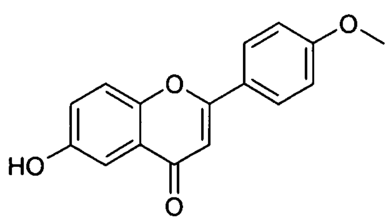

Trockenes, pulverförmiges Lithiumhydroxid (19,7 mmol, 3 Äquivalente) wird auf einmal zu einer gut gerührten Lösung von 2',5'dihydroxyacetophenon (6,4 mmol) in trockenem THF (5 ml) unter Argonatmosphäre bei - 78°C zugegeben. Die Reaktionsmischung wird bei — 78°C während einer Stunde gerührt und anschließend während zwei stunden bei -10°C. Nach erneutem Abkühlen auf —78°C, wird auf einmal eine Lösung von 4-Methoxybenzoesäurechlorid (6,5 mmol) in THF (10 ml) zugegeben. Während einer Stunde wird bei -78°C und bei Raumtemperatur während 4 Stunden, bis zum Verschwinden des Ausgangmaterials gerührt. Die Reaktionsmischung wird auf eine Mischung aus Eis (150 g) und konzentriert HCl (5 ml) geleert und mit Dichloromethan (3x50 ml)extrahiert. Die lösungsmittel werden aus den getrockneten Extrakten entfernt und den Rückstand unter Vakuum während 24 Stunden getrocknet. Der Rückstand wird mit Eisessig (30 ml) und Schwefelsäure (0,2 ml) versetzt und unter Argonatmosphäre während 30 Minuten bis zur einer Stunde auf 95-100°C erhitzt. Etwa ein Drittel der Essigsäure wird abgezogen und der Rückstand in Wasser geleert. Das ausgefällte Produkt wird filtriert, gewaschen und getrocknet und in Methanol umkristallisiert um 4'-Methoxy-6-hydroxyflavon zu ergeben. $^1H$ NMR (500 MHz, $d^6$-DMSO): δ 10,08 (br s, OH), 8,02-7,11 (dm, 4H), 7,63 (d, 1H), 7,35 (d, 1H), 7,26 (d, 1H), 6,86 (s, 1H), 3,88 (s, 3H); $^{13}C$ NMR (62,90 MHz, $d^6$-DMSO): δ 176,78 (s), 162,23 (s), 161,93 (s), 154,73 (s), 149,24 (s), 127,97 (s), 124,14 (s), 123,44 (s), 122,77 (s), 119,61 (s), 114,46 (s), 107,52 (s), 104,44 (s), 55,43 (s); EI-MS (70eV) m/z (rel. abund.)= 268,0735 (100). Das Produkt ist farblos und zeigt das UV-Absorptionsmaximum bei λ = 322 nm mit ε = 24790 (in 2-Propanol). (vgl. Figur 1).

**Beispiel 2: Herstellung von 4'-Methoxy-7-hydroxyflavon**

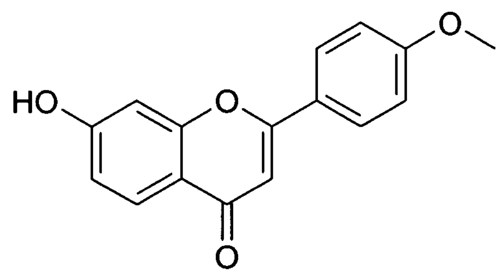

Trockenes, pulverförmiges Lithiumhydroxid (38,7 mmol, 3 Äquivalente) wird auf einmal zu einer gut gerührten Lösung von 2',4'dihydroxyacetophenon (12,9 mmol) in trockenem THF (15 ml) unter Argonatmosphäre bei - 78°C zugegeben. Die Reaktionsmischung wird bei -78°C während einer Stunde gerührt und anschließend während zwei stunden bei -10°C. Nach erneutem Abkühlen auf -78°C, wird auf einmal eine Lösung von 4-Methoxybenzoesäurechlorid (14,2 mmol) in THF (20 ml) zugegeben. Während einer Stunde wird bei -78°C und bei Raumtemperatur während 4 Stunden, bis zum Verschwinden des Ausgangmaterials gerührt. Die Reaktionsmischung wird auf eine Mischung aus Eis (300 g) und konzentriert HCl (10 ml) geleert und mit Dichloromethan (3x50 ml) extrahiert. Die lösungsmittel werden aus den getrockneten Extrakten entfernt und den Rückstand unter Vakuum während 24 Stunden getrocknet. Der Rückstand wird mit Eisessig (100 ml) und Schwefelsäure (0,5 ml) versetzt und unter Argonatmosphäre während 30 Minuten bis zur einer Stunde auf 95-100°C erhitzt. Etwa ein Drittel der Essigsäure wird abgezogen und der Rückstand in Wasser geleert. Das ausgefällte Produkt wird filtriert, gewaschen und getrocknet und in Methanol umkristallisiert um 4'-Methoxy-7-hydroxyflavon zu ergeben. $^1H$ NMR (250 MHz, $d^6$-DMSO): δ 10,90 (br s, OH), 8,01-7,11 (dm, 4H), 7,87 (d, 1H), 7,00 (d, 1H), 6,93 (d, 1H), 6,81 (s, 1H), 3,86 (s, 3H); $^{13}C$ NMR (62,90 MHz, $d^6$-DMSO): δ 176,24 (s), 162,58 (s), 162,00 (s), 161,87 (s), 157,39 (s), 127,93 (s), 126,41 (s), 123,42 (s), 116,07 (s), 114,83 (s), 114,48 (s), 105,07 (s), 102,48 (s), 55,46 (s); EI-MS (70eV) m/z (rel. abund.)= 268,07356 (100). Das Produkt ist beige und zeigt das UV-Absorptionsmaximum bei λ= 323 nm mit ε = 26450 (in 2-Propanol). (vgl. Figur 2);

**Beispiel 3 Herstellung von 4',7-Dihydroxyflavon**

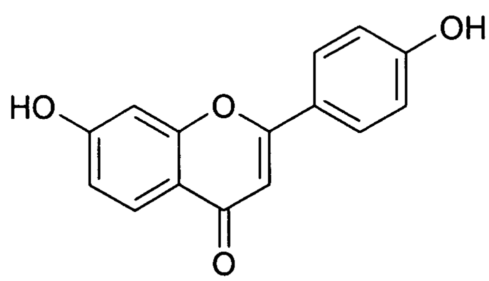

Eine Bortribromid-Lösung (1,8ml, 6 Äquivalent) wird zu einer gut gerührten Lösung von 4'-Methoxy-7-hydroxyflavon (3 mmol) in Dichloromethan (50 ml) unter Argonatmosphäre bei -78°C. Nach dem kompletten Zugabe der Bortribromid-Lösung, wird die Reaktionsmischung bei Raumtemperatur während 24 Stunden und auf einer Mischung aus Eis-Wasser (300 ml). Das Produkt wird filtriert und in Ethanol/Wasser umkristallisiert um 4',7-dihydroxyflavon zu ergeben. $^1H$ NMR (300 MHz, $d^6$-DMSO): δ 10,78 (br s, OH), 10,28 (br s, OH), 7,91-6,93 (dm, 4H), 7,87 (d, 1H), 6,98 (d, 1H), 6,72 (s, 1H; $^{13}C$ NMR (62,90 MHz, $d^6$-DMSO): δ 176,19 (s), 162,43 (s), 162,33 (s), 160,58 (s), 157,27 (s), 128,02 (s), 126,36 (s), 121,68 (s), 116,01 (s), 115,78 (s), 114,67 (s), 104,37(s), 102,48 (s); EI-MS (70eV) m/z (rel. abund.)= 254,24 (100). Das Produkt ist gelblich und zeigt das UV-Absorptionsmaximum bei λ = 329 nm mit ε = 27960 (in 2-Propanol). (vgl. Figur 3);

**Beispiel 4 Herstellung von 4'-Methoxy-7-ß-glucosidflavon**

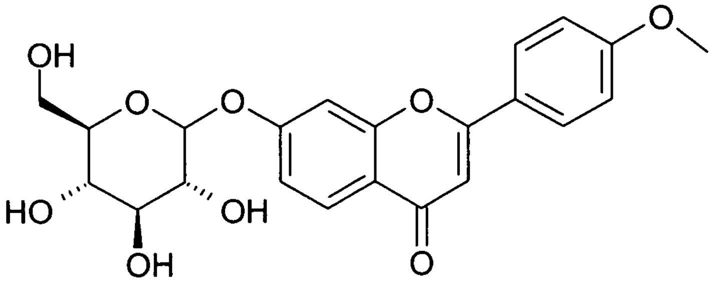

Das 4'-Methoxy-7-hydroxyflavon (3,7 mmol) wird in Natronlauge (50 ml) bei Raumtemperatur während 15 Minuten gerührt. Das D-(+)-Alpha-Acetobromglucose (11 mmol) wird mit dem Tetra-n-butylammoniumbromid (5,6 mmol) in Dichloromethan gelöst und zügig zu der Reaktionsmischung gegeben. Die Reaktionsmischung wird heftig bei Raumtemperatur während 5 Stunden gerührt und mit Schwefelsäure und Wasser neutralisiert. Das Produkt wird mit Essigsäureethylester extrahiert und die organischen Phasen werden mit Wasser und einer NaCI-Lösung nachgewaschen, und die Lösungsmittel werden aus den getrockneten Extrakten entfernt um ein gelber Kristallbrei zu ergeben. Der Rückstand (2,3 g) wird in einer Lösung aus Natriummethylat (18,5 mmol) in Methanol (150 ml) gelöst und bei Raumtemperatur über Nacht gerührt. Das ausgefällte Produkt wird mit Eisessig neutralisiert , PH. Ca. 5-6, und im Eisbad während einer Stunde eingekühlt. Der Feststoff wird filtriert und mit Methanol und Hexan nachgewaschen und über Nacht im Vakuumschrank bei 40°C und 200 mbar getrocknet um 4'-Methoxy-7-ß-glucosidflavon (643 mg, 97,7% rein) zu ergeben. $^1H$ NMR (500 MHz, $d^{6-}$DMSO): δ 8,05-7,12 (dm, 4H), 7,95 (d, 1H), 7,37 (d, 1H), 7,12 (d, 1H), 6,88 (s, 1H), 5,41 (d, OH), 5,12 (m, OH+1H), 5,05 (d, OH), 4,58 (d, OH), 3,87 (s, 3H), 3,74 (m, 1H), 3,51 (m, 2H), 3,34 (m, 2H), 3,23 (m, 1H); EI-MS (70eV) m/z (rel. abund.)= 430,1264 (100). Das Produkt ist leicht gelblich und zeigt das UV-Absorptionsmaximum bei λ = 318 nm mit ε = 37185 (in 2-Propanol). (vgl. Figur 4);

**Beispiel 5: 2-(3',4',5'-Trimethoxyphenyl)-6,7-dihydroxy-4-oxo-4H-1-benzopyran**

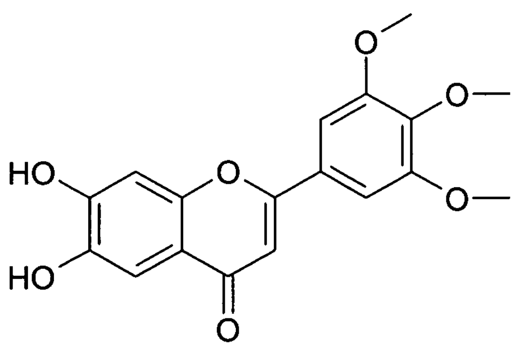

Die Verbindung wird entsprechend Beispiel 2 hergestellt.

13C NMR (DMSO-$d_6$, 300 MHz) δ 176.18 (C-4), 161.21 (C-2), 153.14 (C-3' and C-5'), 152.19 (C-7), 150.72 (C-9), 144.52 (C-6), 140.05 (C-4'), 126.90 (C-1'), 115.99 (C-10), 107.46 (C-5), 105.82 (C-8), 103.66 (C-2' and C-6'), 103.29 (C-3), 60.12 (OCH3 on C-4'), 56.18 (OCH3 on C-3' and C-5').

EI-MS m/z (% relative abundance) composition: 344.0896 (100), 343 (1), 330 (8), 329 (31), 328 (1), 315 (7), 314 (6), 302 (2), 301 (8), 286 (2), 284 (2), 273 (5), 271 (4), 269 (3), 258 (2), 255 (1), 243 (3), 242 (2), 241 (4), 215 (2), 213 (1), 195 (1), 193 (1), 192 (8), 187 (4), 177 (11), 162 (2), 158 (8), 153 (9), 152 (4), 149 (6), 143 (7) 137 (2), 135 (3), 134 (4), 132 (2), 128 (4), 121 (2), 119 (5), 117 (2), 115 (3), 107 (3), 106 (2), 101 (1), 100 (1).

UV-vis (2-propanol, 1 mg / 100 mL) $\lambda_{max}$ (e) nm: 281 (8267), 324 (16036);

Anal. Calcd for C18H16O7: C, 62.79; H, 4.68; O, 32.53. Found: C, H, O,.

**Beispiel 6: 2-(4'-Hydroxyphenyl)-6-hydroxy-4-oxo-4H-1-benzopyran**

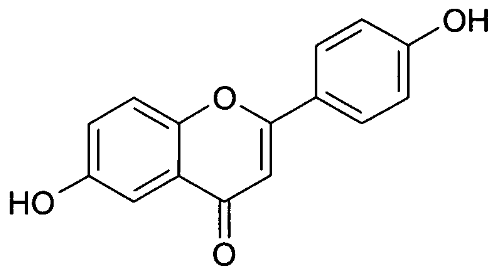

Die Verbindung wird entsprechend Beispiel 3 hergestellt.

1H NMR (DMSO-$d_6$, 300 MHz) δ 10.17 (br s, 2H, exchanges with D20, OH on C-6 and C-4'), 7.4251 (AB, 4H, dA = 7.91183 (H-3' and H-5') and dB = 6.9318 (H-2' and H-6'), JAB = 9.5229), 7.61 (d, 1H, 3J8, 7 = 9.52, H-8), 7.34 (d, 1H, 4J5, 7 = 2.64, H-5), 7.18 (dd, 1H, 3J7, 8 = 9.52, 4J7, 5 = 2.64, H-7), 6.79 (s, 1H, H-3).

13C NMR (DMSO-d6, 75.47 MHz) d 176.70 (C-4), 162.59 (C-2), 160.68 (C-4'), 154.62 (C-6), 149.14 (C-9), 128.10 (C-2' and C-6'), 124.10 (C-10), 122.62 (C-7), 121.74 (C-1'), 119.53 (C-8), 115.81 (C-3' and C-5'), 107.45 (C-5), 103.80 (C-3).

El-MS m/z (% relative abundance) composition: 254.0579 (100), 253 (13), 237 (2), 226 (5), 225 (3), 197 (3), 139 (2), 137 (35), 136 (78), 135 (4), 121 (2), 119 (4), 118 (14), 113 (9), 108 (12), 107 (3), 99 (1), 89 (5), 86 (1), 82 (4), 80 (10), 76 (1), 65 (2), 63 (6), 54 (2), 52 (11), 39 (3).

UV-vis (2-propanol, 1 mg / 100 mL) $\lambda_{max}$ (e) nm: 228 (23068), 277 (14805), 328(30971);

Anal. Calcd for C15H10O4 C, 70.86%; H, 3.96%; O, 25.17%. Found: C, 70.4%; H, 4.0%; O, 25.4%.

Im folgenden werden beispielhaft Rezepturen für kosmetische Zubereitungen angegeben, die Verbindungen nach Beispielen 1 - 4 enthalten. Im übrigen sind die INCI-Bezeichnungen der handelsüblichen Verbindungen angegeben.

UV-Pearl, OMC steht für die Zubereitung mit der INCI-Bezeichnung:

Water (for EU: Aqua), Ethylhexyl Methoxycinnamate, Silica, PVP, Chlorphenesin, BHT; diese Zubereitung ist im handel unter der Bezeichnung Eusolex®UV Pearl™OMC von der Merck KGaA, Darmstadt erhältlich.

Die anderen in den Tabellen angegebenen UV-Pearl sind jeweils analog zusammengesetzt, wobei OMC gegen die angegebenen UV-Filter ausgetauscht wurde.

Tabelle 1 W/O-Emulsionen (Zahlen in Gew.-%)

| | 1-1 | 1-2 | 1-3 | 1-4 | 1-5 | 1-6 | 1-7 | 1-8 | 1-9 | 1-10 |
|---|---|---|---|---|---|---|---|---|---|---|
| Titanium dioxide | | 2 | 5 | | | | | | | 3 |
| 4'-Methoxy-7-hydroxyflavon | 5 | 3 | 2 | 1 | 2 | | | | 1 | 1 |
| 4'-Methoxy-6-hydroxyflavon | | | | | | 1 | 2 | 1 | | |
| Zinc oxide | | | | | | | | 5 | 2 | |
| UV-Pearl , OMC | 30 | 15 | 15 | 15 | 15 | 15 | 15 | 15 | 15 | 15 |
| Polyglyceryl-3-Dimerate | 3 | 3 | 3 | 3 | 3 | 3 | 3 | 3 | 3 | 3 |
| Cera Alba | 0,3 | 0,3 | 0,3 | 0,3 | 0,3 | 0,3 | 0,3 | 0,3 | 0,3 | 0,3 |
| Hydrogenated Castor Oil | 0,2 | 0,2 | 0,2 | 0,2 | 0,2 | 0,2 | 0,2 | 0,2 | 0,2 | 0,2 |
| Paraffinium Liquidum | 7 | 7 | 7 | 7 | 7 | 7 | 7 | 7 | 7 | 7 |
| Caprylic/Capric Triglyceride | 7 | 7 | 7 | 7 | 7 | 7 | 7 | 7 | 7 | 7 |
| Hexyl Laurate | 4 | 4 | 4 | 4 | 4 | 4 | 4 | 4 | 4 | 4 |
| PVP/Eicosene Copolymer | 2 | 2 | 2 | 2 | 2 | 2 | 2 | 2 | 2 | 2 |
| Propylene Glycol | 4 | 4 | 4 | 4 | 4 | 4 | 4 | 4 | 4 | 4 |
| Magnesium Sulfate | 0,6 | 0,6 | 0,6 | 0,6 | 0,6 | 0,6 | 0,6 | 0,6 | 0,6 | 0,6 |
| Tocopherol | 0,5 | 0,5 | 0,5 | 0,5 | 0,5 | 0,5 | 0,5 | 0,5 | 0,5 | 0,5 |
| Tocopheryl Acetate | 0,5 | 0,5 | 0,5 | 0,5 | 0,5 | 0,5 | 0,5 | 0,5 | 0,5 | 0,5 |
| Cyclomethicone | 0,5 | 0,5 | 0,5 | 0,5 | 0,5 | 0,5 | 0,5 | 0,5 | 0,5 | 0,5 |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Water | ad 100 | ad 100 | ad 100 | ad 100 | ad 100 | ad 100 | ad 100 | ad 100 | ad 100 | ad 100 |

Tabelle 1 (Fortsetzung)

| | 1-11 | 1-12 | 1-13 | 1-14 | 1-15 | 1-16 | 1-17 | 1-18 |
|---|---|---|---|---|---|---|---|---|
| Titanium dioxide | 3 | | 2 | | 3 | | 2 | 5 |
| Benzylidene malonate polysiloxane | | 1 | 0,5 | | | | | |
| Methylene Bis-Benztriazolyl Tetramethylbutylphenol | 1 | 1 | 0,5 | | | | | |
| 4',7-Dihydroxyflavon | 5 | 3 | 2 | 5 | 1 | 3 | 7 | 2 |
| Polyglyceryl-3-Dimerate | 3 | 3 | 3 | 3 | | | | |
| Cera Alba | 0,3 | 0,3 | 0,3 | 0,3 | 2 | 2 | 2 | 2 |
| Hydrogenated Castor Oil | 0,2 | 0,2 | 0,2 | 0,2 | | | | |
| Paraffinium Liquidum | 7 | 7 | 7 | 7 | | | | |
| Caprylic/Capric Triglyceride | 7 | 7 | 7 | 7 | | | | |
| Hexyl Laurate | 4 | 4 | 4 | 4 | | | | |
| PVP/Eicosene Copolymer | 2 | 2 | 2 | 2 | | | | |
| Propylene Glycol | 4 | 4 | 4 | 4 | | | | |
| Magnesium Sulfate | 0,6 | 0,6 | 0,6 | 0,6 | | | | |
| Tocopherol | 0,5 | 0,5 | 0,5 | 0,5 | | | | |
| Tocopheryl Acetate | 0,5 | 0,5 | 0,5 | 0,5 | 1 | 1 | 1 | 1 |
| Cyclomethicone | 0,5 | 0,5 | 0,5 | 0,5 | | | | |
| Propylparabene | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 |
| Dicocoyl Pentyerythrityl Citrate (and) Sorbitan Sesquioleate (and) Cera Alba (and) Aluminium Stearate | | | | | 6 | 6 | 6 | 6 |
| PEG-7 Hydrogenated Castor Oil | | | | | 1 | 1 | 1 | 1 |
| Zinc Stearate | | | | | 2 | 2 | 2 | 2 |
| Oleyl Erucate | | | | | 6 | 6 | 6 | 6 |
| Decyl Oleate | | | | | 6 | 6 | 6 | 6 |
| Dimethicone | | | | | 5 | 5 | 5 | 5 |
| Tromethamine | | | | | 1 | 1 | 1 | 1 |
| Glycerin | | | | | 5 | 5 | 5 | 5 |
| Allantoin | | | | | 0,2 | 0,2 | 0,2 | 0,2 |
| water | ad 100 | ad 100 | ad 100 | ad 100 | ad 100 | ad 100 | ad 100 | ad 100 |

Tabelle 1 (Fortsetzung)

| | 1-19 | 1-20 | 1-21 | 1-22 | 1-23 | 1-24 | 1-25 | 1-26 | 1-27 | 1-28 | 1-29 |
|---|---|---|---|---|---|---|---|---|---|---|---|
| Titanium dioxide | | 2 | 5 | | | | | | | 3 | 3 |
| Benzylidene malonate polysiloxane | | | | 1 | | | | | 1 | 1 | |
| Methylene Bis-Benztriazolyl Tetramethylbutylphenol | | | | | | 1 | 2 | 1 | | | 1 |
| Zinc oxide | | | | | | | | 5 | 2 | | |
| UV-Pearl OMC | 5 | 5 | 5 | 5 | 7 | 5 | 5 | 5 | 5 | 5 | 8 |
| UV-Pearl , OCR | | 10 | | | | | | | | | 5 |
| UV-Pearl, EthylhexylDimethylPABA | | | 10 | | | | | | | | |
| UV-Pearl, Homosalate | | | | 10 | | | | | | | |
| UV-Pearl, Ethylhexyl salicylate | | | | | 10 | | | | | | |
| UV-Pearl , OMC, BP-3 | | | | | | 10 | | | | | |
| UV-Pearl , OCR, BP-3 | | | | | | | 10 | | | | |
| UV-Pearl, Ethylhexyl Dimethyl PABA, BP-3 | | | | | | | | 10 | | | |
| UV-Pearl, Homosalate, BP-3 | | | | | | | | | 10 | | |
| UV-Pearl, Ethylhexyl salicylate, BP-3 | | | | | | | | | | 10 | |
| BMDBM | | | | | | | | | | | 2 |
| UV-Pearl OMC, 4-Methylbenzylidene Camphor | 25 | | | | | | | | | | |
| Polyglyceryl-3-Dimerate | 3 | 3 | 3 | 3 | 3 | 3 | 3 | 3 | 3 | 3 | 3 |
| Cera Alba | 0,3 | 0,3 | 0,3 | 0,3 | 0,3 | 0,3 | 0,3 | 0,3 | 0,3 | 0,3 | 0,3 |
| Hydrogenated Castor Oil | 0,2 | 0,2 | 0,2 | 0,2 | 0,2 | 0,2 | 0,2 | 0,2 | 0,2 | 0,2 | 0,2 |
| Paraffinium Liquidum | 7 | 7 | 7 | 7 | 7 | 7 | 7 | 7 | 7 | 7 | 7 |
| Caprylic/Capric Triglyceride | 7 | 7 | 7 | 7 | 7 | 7 | 7 | 7 | 7 | 7 | 7 |
| Hexyl Laurate | 4 | 4 | 4 | 4 | 4 | 4 | 4 | 4 | 4 | 4 | 4 |
| PVP/Eicosene Copolymer | 2 | 2 | 2 | 2 | 2 | 2 | 2 | 2 | 2 | 2 | 2 |
| Propylene Glycol | 4 | 4 | 4 | 4 | 4 | 4 | 4 | 4 | 4 | 4 | 4 |
| Magnesium Sulfate | 0,6 | 0,6 | 0,6 | 0,6 | 0,6 | 0,6 | 0,6 | 0,6 | 0,6 | 0,6 | 0,6 |
| Tocopherol | 0,5 | 0,5 | 0,5 | 0,5 | 0,5 | 0,5 | 0,5 | 0,5 | 0,5 | 0,5 | 0,5 |
| Tocopheryl Acetate | 0,5 | 0,5 | 0,5 | 0,5 | 0,5 | 0,5 | 0,5 | 0,5 | 0,5 | 0,5 | 0,5 |
| Cyclomethicone | 0,5 | 0,5 | 0,5 | 0,5 | 0,5 | 0,5 | 0,5 | 0,5 | 0,5 | 0,5 | 0,5 |
| Propylparabene | 0,05 | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 | 0,15 |
| Water | ad 100 | | | | | | | | | | |

Tabelle 2: O/W-Emulsionen, Zahlen in Gew.-%

| | 2-1 | 2-2 | 2-3 | 2-4 | 2-5 | 2-6 | 2-7 | 2-8 | 2-9 | 2-10 |
|---|---|---|---|---|---|---|---|---|---|---|
| Titanium dioxide | | 2 | 5 | | | | | | | 3 |
| Methylene Bis-Benztriazolyl Tetramethylbutylphenol | | | | | | 1 | 2 | 1 | | |
| 4´-Methoxy-7-ß-glucosidflavon | | | | 1 | 2 | | | | 1 | 1 |
| 4‘-Methoxy-6-hydroxyflavon | 1 | 3 | | 2 | | 5 | | 5 | 2 | |
| 4´-Methoxy-7-hydroxyflavon | 5 | 5 | 5 | 5 | 5 | 5 | 5 | 5 | 5 | 5 |
| 4´,7-Dihydroxyflavon | 1 | 5 | 4 | | 6 | | 7 | | 2 | 1 |
| 4-Methylbenzyliden Camphor | 2 | | 3 | | 4 | | 3 | | 2 | |
| BMDBM | 1 | 3 | | 3 | 3 | | 3 | 3 | 3 | |
| Stearyl Alcohol (and) Steareth-7 (and) Steareth-10 | 3 | 3 | 3 | 3 | 3 | 3 | 3 | 3 | 3 | 3 |
| Glyceryl Stearate (and) Ceteth-20 | 3 | 3 | 3 | 3 | 3 | 3 | 3 | 3 | 3 | 3 |
| Glyceryl Stearate | 3 | 3 | 3 | 3 | 3 | 3 | 3 | 3 | 3 | 3 |
| Microwax | 1 | 1 | 1 | 1 | 1 | 1 | 1 | 1 | 1 | 1 |
| Cetearyl Octanoate | 11,5 | 11,5 | 11,5 | 11,5 | 11,5 | 11,5 | 11,5 | 11,5 | 11,5 | 11,5 |
| Caprylic/Capric Triglyceride | 6 | 6 | 6 | 6 | 6 | 6 | 6 | 6 | 6 | 6 |
| Oleyl Oleate | 6 | 6 | 6 | 6 | 6 | 6 | 6 | 6 | 6 | 6 |
| Propylene Glycol | 4 | 4 | 4 | 4 | 4 | 4 | 4 | 4 | 4 | 4 |
| Glyceryl Stearate SE | | | | | | | | | | |
| Stearic Acid | | | | | | | | | | |
| Persea Gratissima | | | | | | | | | | |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Tromethamine | | | 1,8 | | | | | | | |
| Glycerin | | | | | | | | | | |
| Water | ad 100 | ad 100 | ad 100 | ad 100 | ad 100 | ad 100 | ad 100 | ad 100 | ad 100 | ad 100 |
| | | | | | | | | | | |

Tabelle 2 (Fortsetzung)

| | 2-11 | 2-12 | 2-13 | 2-14 | 2-15 | 2-16 | 2-17 | 2-18 |
|---|---|---|---|---|---|---|---|---|
| Titanium dioxide | 3 | | 2 | | | | 2 | 5 |
| Benzylidene malonate polysiloxane | | 1 | 0,5 | | | | | |
| Methylene Bis-Benztriazolyl Tetramethylbutylphenol | 1 | 1 | 0,5 | | | | | |
| 4′-Methoxy-7-ß-glucosidflavon | | | | 1 | 2 | | | |
| 4'-Methoxy-6-hydroxyflavon | 1 | 3 | | 2 | | 5 | | 5 |
| 4′-Methoxy-7-hydroxyflavon | 5 | 5 | 5 | 5 | 5 | 5 | 5 | 5 |
| 4′,7-Dihydroxyflavon | 1 | 5 | 4 | | 6 | | 7 | |
| Zinc oxide | | | 2 | | | | | |
| UV-Pearl , OMC | 15 | 15 | 15 | 30 | 30 | 30 | 15 | 15 |
| 4-Methylbenzyliden Camphor | | | | 3 | | | | |
| BMDBM | | | | 1 | | | | |
| Phenylbenzimidazole Sulfonic Acid | | | | | 4 | | | |
| Stearyl Alcohol (and) Steareth-7 (and) Steareth-10 | 3 | 3 | 3 | 3 | | | | |
| Glyceryl Stearate (and) Ceteth-20 | 3 | 3 | 3 | 3 | | | | |
| Glyceryl Stearate | 3 | 3 | 3 | 3 | | | | |
| Microwax | 1 | 1 | 1 | 1 | | | | |
| Cetearyl Octanoate | 11,5 | 11,5 | 11,5 | 11,5 | | | | |
| Caprylic/Capric Triglyceride | 6 | 6 | 6 | 6 | 14 | 14 | 14 | 14 |
| Oleyl Oleate | 6 | 6 | 6 | 6 | | | | |
| Propylene Glycol | 4 | 4 | 4 | 4 | | | | |
| Glyceryl Stearate SE | | | | | 6 | 6 | 6 | 6 |
| Stearic Acid | | | | | 2 | 2 | 2 | 2 |
| Persea Gratissima | | | | | 8 | 8 | 8 | 8 |
| Propylparabene | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 |
| Tromethamine | | | | | 1,8 | | | |
| Glycerin | | | | | 3 | 3 | 3 | 3 |
| Water | ad 100 | ad 100 | ad 100 | ad 100 | ad 100 | ad 100 | ad 100 | ad 100 |

Tabelle 2 (Fortsetzung)

| | 2-19 | 2-20 | 2-21 | 2-22 | 2-23 | 2-24 | 2-25 | 2-26 | 2-27 | 2-28 |
|---|---|---|---|---|---|---|---|---|---|---|
| Titanium dioxide | | | | | | | 3 | 3 | | 2 |
| Benzylidene malonate polysiloxane | 1 | 2 | | | | 1 | 1 | | 1 | 0,5 |
| 4'-Methoxy-7-ß-glucosidflavon | | | | 1 | 2 | | | | 1 | 1 |
| 4'-Methoxy-6-hydroxyflavon | 1 | 3 | | 2 | | 5 | | 5 | 2 | |
| 4'-Methoxy-7-hydroxyflavon | 5 | 5 | 5 | 5 | 5 | 5 | 5 | 5 | 5 | 5 |
| 4',7-Dihydroxyflavon | 1 | 5 | 4 | | 6 | | 7 | | 2 | 1 |
| Methylene Bis-Benztriazolyl Tetramethylbutylphenol | | | 1 | 2 | 1 | | | 1 | 1 | 0,5 |
| Zinc oxide | | | | | 5 | 2 | | | | 2 |
| UV-Pearl , OMC | 15 | 15 | 15 | 15 | 15 | 15 | 15 | 15 | 15 | 15 |
| Caprylic/Capric Triglyceride | 14 | 14 | 14 | 14 | 14 | 14 | 14 | 14 | 14 | 14 |
| Oleyl Oleate | | | | | | | | | | |
| Propylene Glycol | | | | | | | | | | |
| Glyceryl Stearate SE | 6 | 6 | 6 | 6 | 6 | 6 | 6 | 6 | 6 | 6 |
| Stearic Acid | 2 | 2 | 2 | 2 | 2 | 2 | 2 | 2 | 2 | 2 |
| Persea Gratissima | 8 | 8 | 8 | 8 | 8 | 8 | 8 | 8 | 8 | 8 |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Glyceryl Stearate, Ceteareth-20, Ceteareth-10, Cetearyl Alcohol, Cetyl Palmitate | | | | | | | | | | |
| Ceteareth-30 | | | | | | | | | | |
| Dicaprylyl Ether | | | | | | | | | | |
| Hexyldecanol, Hexyldexyllaurate | | | | | | | | | | |
| Cocoglycerides | | | | | | | | | | |
| Tromethamine | | | | | | | | | | |
| Glycerin | 3 | 3 | 3 | 3 | 3 | 3 | 3 | 3 | 3 | 3 |
| Water | ad 100 | ad 100 | ad 100 | ad 100 | ad 100 | ad 100 | ad 100 | ad 100 | ad 100 | ad 100 |

Tabelle 3: Gele, Zahlen in Gew.-%

| | 3-1 | 3-2 | 3-3 | 3-4 | 3-5 | 3-6 | 3-7 | 3-8 | 3-9 | 3-10 |
|---|---|---|---|---|---|---|---|---|---|---|
| a = aqueaous gel | | | | | | | | | | |
| Titanium dioxide | | 2 | 5 | | | | | | | 3 |
| 4'-Methoxy-7-ß-glucosidflavon | | | | 1 | 2 | | | | 1 | 1 |
| 4'-Methoxy-6-hydroxyflavon | 1 | 3 | | 2 | | 5 | | 5 | 2 | |
| 4'-Methoxy-7-hydroxyflavon | 5 | 5 | 5 | 5 | 5 | 5 | 5 | 5 | 5 | 5 |
| 4',7-Dihydroxyflavon | 1 | 5 | 4 | | 6 | | 7 | | 2 | 1 |
| Benzylidene malonate polysiloxane | | | 1 | 1 | 2 | | | | 1 | 1 |
| Methylene Bis-Benztriazolyl Tetramethylbutylphenol | | 1 | | | | 1 | 2 | 1 | | |
| Zinc oxide | | | | 2 | | | | 5 | 2 | |
| UV-Pearl , Ethylhexyl Mehtoxycinnamat | 30 | 15 | 15 | 15 | 15 | 15 | 15 | 15 | 15 | 15 |
| 4-Methylbenzyliden Camphor | | | | | 2 | | | | | |
| Butylmethoxydibenzoylmethane | | 1 | | | | | | | | |
| Phenylbenzimidazole Sulfonic Acid | | | 4 | | | | | | | |
| Prunus Dulcis | 5 | 5 | 5 | 5 | 5 | 5 | 5 | 5 | 5 | 5 |
| Tocopheryl Acetate | 0,5 | 0,5 | 0,5 | 0,5 | 0,5 | 0,5 | 0,5 | 0,5 | 0,5 | 0,5 |
| Caprylic/Capric Triglyceride | 3 | 3 | 3 | 3 | 3 | 3 | 3 | 3 | 3 | 3 |
| Octyldodecanol | 2 | 2 | 2 | 2 | 2 | 2 | 2 | 2 | 2 | 2 |
| Decyl Oleate | 2 | 2 | 2 | 2 | 2 | 2 | 2 | 2 | 2 | 2 |
| PEG-8 (and) Tocopherol (and) Ascorbyl Palmitate (and) Ascorbic Acid (and) Citric Acid | 0,05 | 0,05 | 0,05 | 0,05 | 0,05 | 0,05 | 0,05 | 0,05 | 0,05 | 0,05 |
| Sorbitol | 4 | 4 | 4 | 4 | 4 | 4 | 4 | 4 | 4 | 4 |
| Polyacrylamide (and) C13-14 Isoparaffin (and) Laureth-7 | 3 | 3 | 3 | 3 | 3 | 3 | 3 | 3 | 3 | 3 |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Tromethamine | | | 1,8 | | | | | | | |
| Water | ad 100 | ad 100 | ad 100 | ad 100 | ad 100 | ad 100 | ad 100 | ad 100 | ad 100 | ad 100 |

Tabelle 3 (Fortsetzung)

| | 3-11 | 3-12 | 3-13 | 3-14 | 3-15 | 3-16 | 3-17 | 3-18 |
|---|---|---|---|---|---|---|---|---|
| a = aqueaous gel | | | | a | a | a | a | a |
| Titanium dioxide | 3 | | 2 | | | | | |
| Benzylidene malonate polysiloxane | | 1 | 0,5 | 1 | 2 | | | |
| Methylene Bis-Benztriazolyl Tetramethylbutylphenol | 1 | 1 | 0,5 | | | 1 | 2 | 1 |
| 4'-Methoxy-7-ß-glucosidflavon | | | | 1 | 2 | | | |
| 4'-Methoxy-6-hydroxyflavon | 1 | 3 | | 2 | | 5 | | 5 |
| 4'-Methoxy-7-hydroxyflavon | 5 | 5 | 5 | 5 | 5 | 5 | 5 | 5 |
| 4',7-Dihydroxyflavon | 1 | 5 | 4 | | 6 | | 7 | |
| Zinc oxide | | | 2 | | | | | |
| UV-Pearl , Ethylhexyl Mehtoxycinnamat | 15 | 15 | 15 | 15 | 15 | 15 | 15 | 15 |
| Prunus Dulcis | 5 | 5 | 5 | | | | | |
| Tocopheryl Acetate | 0,5 | 0,5 | 0,5 | | | | | |
| Caprylic/Capric Triglyceride | 3 | 3 | 3 | | | | | |
| Octyldodecanol | 2 | 2 | 2 | | | | | |
| Decyl Oleate | 2 | 2 | 2 | | | | | |
| PEG-8 (and) Tocopherol (and) Ascorbyl Palmitate (and) Ascorbic Acid (and) Citric Acid | 0,05 | 0,05 | 0,05 | | | | | |
| Sorbitol | 4 | 4 | 4 | 5 | 5 | 5 | 5 | 5 |
| Polyacrylamide (and) C13-14 Isoparaffin (and) Laureth-7 | 3 | 3 | 3 | | | | | |
| Carbomer | | | | 1,5 | 1,5 | 1,5 | 1,5 | 1,5 |
| Propylparabene | 0,05 | 0,05 | 0,05 | | | | | |
| Methylparabene | 0,15 | 0,15 | 0,15 | 0,15 | 0,15 | 0,15 | 0,15 | 0,15 |
| Allantoin | | | | 0,2 | 0,2 | 0,2 | 0,2 | 0,2 |
| Tromethamine | | | | 2,4 | 2,4 | 2,4 | 2,4 | 2,4 |
| Water | ad 100 | ad 100 | ad 100 | ad 100 | ad 100 | ad 100 | ad 100 | ad 100 |

Tabelle 3 (Fortsetzung)

| | 3-19 | 3-20 | 3-21 | 3-22 | 3-23 | 3-24 | 3-25 | 3-26 | 3-27 | 3-28 |
|---|---|---|---|---|---|---|---|---|---|---|
| 4´-Methoxy-7-ß-glucosidflavon | | | | 1 | 2 | | | | 1 | 1 |
| 4'-Methoxy-6-hydroxyflavon | 1 | 3 | | 2 | | 5 | | 5 | 2 | |
| 4´-Methoxy-7-hydroxyflavon | 5 | 5 | 5 | 5 | 5 | 5 | 5 | 5 | 5 | 5 |
| 4´,7-Dihydroxyflavon | 1 | 5 | 4 | | 6 | | 7 | | 2 | 1 |
| UV-Pearl , OMC | 30 | 30 | 15 | 15 | 15 | 11 | 12 | 15 | 15 | 15 |
| Phenylbenzimidazole Sulfonic Acid | | 4 | 4 | | | | | | | |
| Sorbitol | 5 | 5 | 5 | 5 | 5 | 5 | 5 | 5 | 5 | 5 |
| Carbomer | 1,5 | 1,5 | 1,5 | 1,5 | 1,5 | 1,5 | 1,5 | 1,5 | 1,5 | 1,5 |
| Propylparabene | | | | | | | | | | |
| Methylparabene | 0,15 | 0,15 | 0,15 | 0,15 | 0,15 | 0,15 | 0,15 | 0,15 | 0,15 | 0,15 |
| Allantoin | 0,2 | 0,2 | 0,2 | 0,2 | 0,2 | 0,2 | 0,2 | 0,2 | 0,2 | 0,2 |
| Tromethamine | 2,4 | 4,2 | 4,2 | 2,4 | 2,4 | 2,4 | 2,4 | 2,4 | 2,4 | 2,4 |
| Water | ad 100 | ad 100 | ad 100 | ad 100 | ad 100 | ad 100 | ad 100 | ad 100 | ad 100 | ad 100 |

Tabelle 3 (Fortsetzung)

| | 3-29 | 3-30 | 3-31 | 3-32 | 3-33 | 3-34 | 3-35 | 3-36 |
|---|---|---|---|---|---|---|---|---|
| 4'-Methoxy-7-ß-glucosidflavon | | | | 1 | 2 | | | |
| 4'-Methoxy-6-hydroxyflavon | 1 | 3 | | 2 | | 5 | | 5 |
| 4'-Methoxy-7-hydroxyflavon | 5 | 5 | 5 | 5 | 5 | 5 | 5 | 5 |
| 4',7-Dihydroxyflavon | 1 | 5 | 4 | | 6 | | 7 | |
| UV-Pearl , OMC | 15 | 10 | | 10 | 10 | 10 | 15 | 10 |
| UV-Pearl , OCR | | | 10 | | | | | |
| UV-Pearl , OMC, Methylene Bis-Benztriazolyl Tetramethylbutylphenol | | 7 | | 6 | | | | |
| UV-Pearl, Ethylhexyl salicylate, BMDBM | | | 10 | | | | | |
| Disodium Phenyl Dibenzimidazole Tetrasulfonate | | 3 | | | | 3 | | 3 |
| Phenylbenzimidazole Sulfonic Acid | | 2 | | | 2 | 3 | | 3 |
| Prunus Dulcis | 5 | 5 | 5 | | | | | |
| Tocopheryl Acetate | 0,5 | 0,5 | 0,5 | | | | | |
| Caprylic/Capric Triglyceride | 3 | 3 | 3 | | | | | |
| Octyldodecanol | 2 | 2 | 2 | | | | | |
| Decyl Oleate | 2 | 2 | 2 | | | | | |
| PEG-8 (and) Tocopherol (and) Ascorbyl Palmitate (and) Ascorbic Acid (and) Citric Acid | 0,05 | 0,05 | 0,05 | | | | | |
| Sorbitol | 4 | 4 | 4 | 5 | 5 | 5 | 5 | 5 |
| Polyacrylamide (and) C13-14 Isoparaffin (and) Laureth-7 | 3 | 3 | 3 | | | | | |
| Carbomer | | | | 1,5 | 1,5 | 1,5 | 1,5 | 1,5 |
| Propylparabene | 0,05 | 0,05 | 0,05 | | | | | |
| Methylparabene | 0,15 | 0,15 | 0,15 | 0,15 | 0,15 | 0,15 | 0,15 | 0,15 |
| Allantoin | | | | 0,2 | 0,2 | 0,2 | 0,2 | 0,2 |
| Tromethamine | | | | 2,4 | 2,4 | 2,4 | 2,4 | 2,4 |
| Water | ad 100 | ad 100 | ad 100 | ad 100 | ad 100 | ad 100 | ad 100 | ad 100 |

**Abbildungen**

**Figur 1:** UV-Absorptionsspektrum von 4'-Methoxy-6-hydroxyflavon
Das UV-VIS-Spektrum wurde auf dem Gerät Varian Cary 100 Bio Spektrophotometer, ausgerüstet mit einem temperierten Küvettenhalter, gemessen (Konzentration = 0,001g/100 ml 2-Propanol; Schichtdicke d = 1cm; Wellenlängenbereich: 200 bis 800 nm).

**Figur 2:** UV-Absorptionsspektrum von 4'-Methoxy-7-hydroxyflavon.
Das UV-VIS-Spektrum wurde auf dem Gerät Varian Cary 100 Bio Spektrophotometer, ausgerüstet mit einem temperierten Küvettenhalter, gemessen (Konzentration = 0,001 g/100 ml 2-Propanol; Schichtdicke d = 1cm; Wellenlängenbereich: 200 bis 800 nm).

**Figur 3:** UV-Absorptionsspektrum von 4', 7-Dihydroxyflavon
Das UV-VIS-Spektrum wurde auf dem Gerät Varian Cary 100 Bio Spektrophotometer, ausgerüstet mit einem temperierten Küvettenhalter, gemessen (Konzentration = 0,001 g/100 ml 2-Propanol; Schichtdicke d = 1cm; Wellenlängenbereich: 200 bis 800 nm).

**Figur 4:** UV-Absorptionsspektrum von 4'-Methoxy-7-β-glucosidflavon
Das UV-VIS-Spektrum wurde auf dem Gerät Varian Cary 100 Bio Spektrophotometer, ausgerüstet mit einem temperierten Küvettenhalter, gemessen (Konzentration = 0,001 g/100 ml 2-Propanol; Schichtdicke d = 1cm; Wellenlängenbereich: 200 bis 800 nm).

**Patentansprüche**

1. Zubereitung mit Lichtschutzeigenschaften, enthaltend zumindest eine Verbindung der Formel I

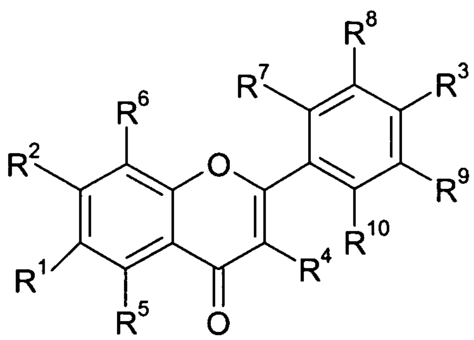

I

wobei $R^1$ und $R^2$ ausgewählt sind aus

- H,
- und $OR^{11}$, wobei $OR^{11}$ unabhängig voneinander steht für
    - OH
    - geradkettige oder verzweigte $C_1$- bis $C_{20}$-Alkyloxygruppen,
    - geradkettigen oder verzweigten $C_3$- bis $C_{20}$-Alkenyloxygruppen,
    - geradkettigen oder verzweigten $C_1$- bis $C_{20}$-Hydroxyalkoxygruppen, wobei die Hydroxygruppe(n) an ein primäre oder sekundäre Kohlenstoffatome der Kette gebunden sein können und weiter die Alkylkette auch durch Sauerstoff unterbrochen sein kann, und/oder
    - $C_3$- bis $C_{10}$-Cycloalkyloxygruppen und/oder $C_3$- bis $C_{12}$-Cycloalkenyloxygruppen, wobei die Ringe jeweils auch durch $-(CH_2)_n$-Gruppen mit n = 1 bis 3 überbrückt sein können und/oder,
    - Mono- und/oder Oligoglycosylreste,

mit der Maßgabe, dass mindestens ein Rest aus $R^1$ und $R^2$ steht für $OR^{11}$,
und $R^3$ steht für einen Rest $OR^{11}$ und
$R^4$ bis $R^7$ und $R^{10}$ gleich oder verschieden sein können, und unabhängig voneinander stehen für

- H
- geradkettige oder verzweigte $C_1$- bis $C_{20}$-Alkylgruppen,
- geradkettige oder verzweigte $C_3$- bis $C_{20}$-Alkenylgruppen,
- geradkettige oder verzweigte $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder
- $C_3$- bis $C_{10}$-Cycloalkylgruppen und/oder $C_3$- bis $C_{12}$-Cycloalkenylgruppen, wobei die Ringe jeweils auch durch $-(CH_2)_n$-Gruppen mit n = 1 bis 3 überbrückt sein können und

$R^8$ und $R^9$ gleich oder verschieden sein können, und unabhängig voneinander stehen für

- H
- $OR^{11}$
- geradkettige oder verzweigte $C_1$- bis $C_{20}$-Alkylgruppen,
- geradkettige oder verzweigte $C_3$- bis $C_{20}$-Alkenylgruppen,
- geradkettige oder verzweigte $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder
- $C_3$- bis $C_{10}$-Cycloalkylgruppen und/oder $C_3$- bis $C_{12}$-Cycloalkenylgruppen, wobei die Ringe jeweils auch durch $-(CH_2)_n$-Gruppen mit n = 1 bis 3 überbrückt sein können.

**2.** Zubereitung mit Lichtschutzeigenschaften nach Anspruch 1, enthaltend zumindest eine Verbindung der Formel I, **dadurch gekennzeichnet, dass** $R^4$ bis $R^7$ und $R^{10}$ H sind.

**3.** Zubereitung mit Lichtschutzeigenschaften nach mindestens einem der vorhergehenden Ansprüche enthaltend zumindest eine Verbindung der Formel I, **dadurch gekennzeichnet, dass** $R^3$ steht für

- OH oder
- geradkettige oder verzweigte $C_1$- bis $C_{20}$-Alkoxygruppen, vorzugsweise Methoxy, Ethoxy oder Ethylhexyloxy oder
- Mono- und/oder Oligoglycosylreste, vorzugsweise Glucosylreste und

$R^1$ und/oder $R^2$ vorzugsweise stehen für

- OH oder
- geradkettige oder verzweigte $C_1$- bis $C_{20}$-Alkoxygruppen, vorzugsweise Methoxy, Ethoxy oder Ethylhexyloxy oder
- Mono- und/oder Oligoglycosylreste, vorzugsweise Glucosylreste.

**4.** Zubereitung mit Lichtschutzeigenschaften nach mindestens einem der vorhergehenden Ansprüche enthaltend zumindest eine Verbindung der Formel I, **dadurch gekennzeichnet, dass** $R^3$ steht für geradkettige oder verzweigte $C_1$- bis $C_{20}$-Alkoxygruppen, vorzugsweise Methoxy, Ethoxy oder Ethylhexyloxy, und $R^8$ und $R^9$ gleich sind und stehen für H oder geradkettige oder verzweigte $C_1$bis $C_{20}$-Alkoxygruppen, vorzugsweise Methoxy, Ethoxy oder Ethylhexyloxy.

**5.** Zubereitung mit Lichtschutzeigenschaften nach mindestens einem der vorhergehenden Ansprüche enthaltend zumindest eine Verbindung der Formel I, **dadurch gekennzeichnet, dass** mindestens einer der Reste $R^1$ bis $R^3$ steht für OH, wobei vorzugsweise mindestens einer der Reste $R^1$ oder $R^2$ für OH steht.

**6.** Zubereitung mit Lichtschutzeigenschaften nach mindestens einem der vorhergehenden Ansprüche, **dadurch gekennzeichnet, dass** eine oder mehrere der Verbindungen gemäß Formel I in verkapselter Form vorliegen.

**7.** Zubereitung mit Lichtschutzeigenschaften nach mindestens einem der vorhergehenden Ansprüche, wobei die Zubereitung neben der mindestens einen Verbindung nach Formel I einen oder mehrere UV-Filter enthält, die vorzugsweise ausgewählt sind aus der Gruppe, die 3-(4'-Methylbenzyliden)-dl-kampfer, 1-(4-tert-Butylphenyl)-3-(4-methoxyphenyl)propan-1,3-dion, 4-Isopropyldibenzoylmethan, 2-Hydroxy-4-methoxybenzophenon, Methoxyzimtsäureoctylester, 3,3,5-Trimethyl-cyclohexylsalicylat, 4-(Dimethylamino)benzoesäure2-ethylhexylester, 2-Cyano-3,3-diphenylacrylsäure-2-ethylhexylester, 2-Phenylbenzimidazol-5-sulfonsäure sowie ihre Kalium-, Natrium- und Triethanolaminsalze enthält.

**8.** Zubereitung mit Lichtschutzeigenschaften nach mindestens einem der vorhergehenden Ansprüche zum Schutz von Körperzellen gegen oxidativen Stress, insbesondere zur Verringerung der Hautalterung, **dadurch gekennzeichnet, dass** sie vorzugsweise ein oder mehrere Antioxidantien enthält.

**9.** Verfahren zur Herstellung einer Zubereitung **dadurch gekennzeichnet, dass** eine Verbindung der Formel I mit Resten gemäß Anspruch 1 mit einem kosmetisch oder dermatologisch geeignetem Träger vermischt wird.

**10.** Verfahren nach Anspruch 8, **dadurch gekennzeichnet, dass** die Verbindung nach Formel I hergestellt wird durch Umsetzung einer 2-Hydroxyacetophenon-Verbindung mit einer Lithiumverbindung und anschließend einer Ketoverbindung.

**11.** Verwendung einer Verbindung der Formel I, wobei die Variablen die in Anspruch 1 angegebene Bedeutung aufweisen, zur Herstellung einer Zubereitung mit Lichtschutzeigenschaften.

**12.** Verwendung einer Verbindung der Formel I, wobei die Variablen die in Anspruch 1 angegebene Bedeutung aufweisen, als UV-Filter.

**13.** Verwendung von Verbindungen der Formel I, wobei alle Reste die in Anspruch 1 angegebene Bedeutung aufweisen, zur Stabilisierung von UV-Filtern, insbesondere Dibenzoylmethan und Derivaten des Dibenzoylmethans.

Fig. 1

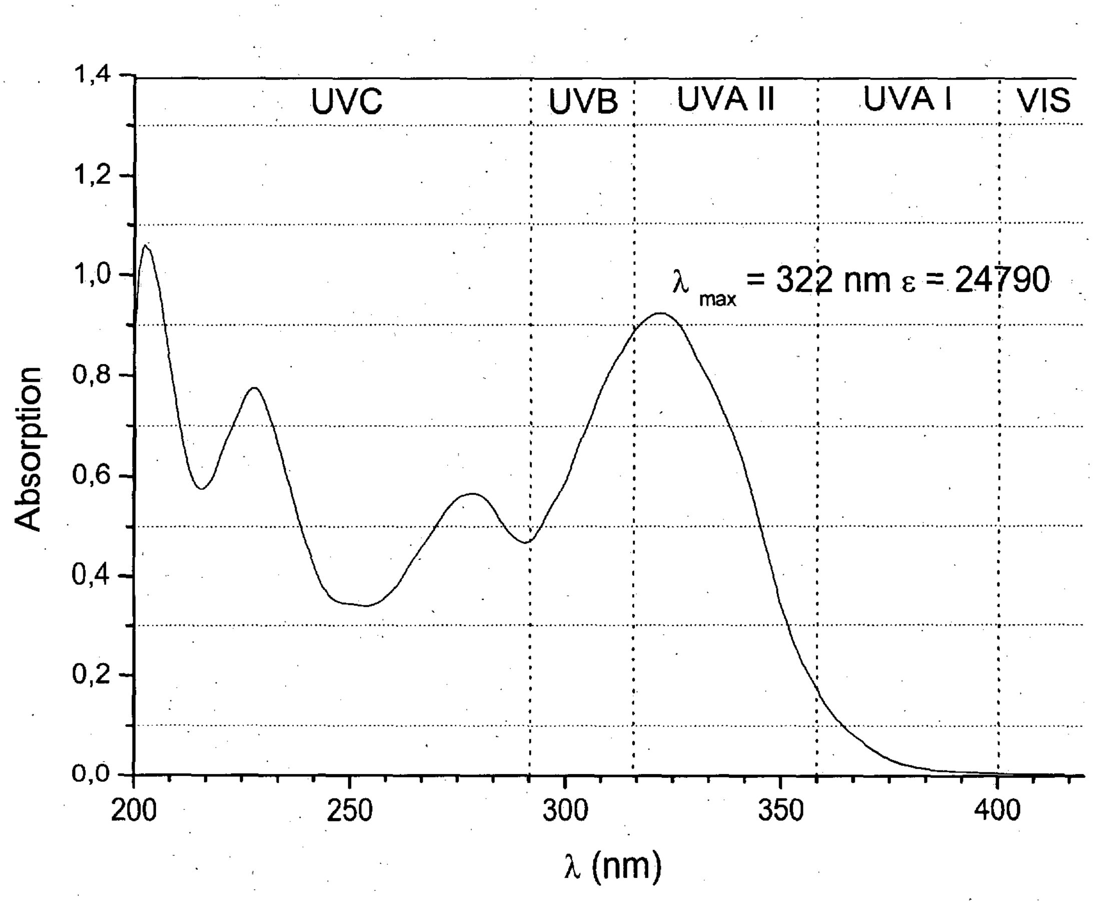
UVC
UVB
UVA II
UVA I
VIS
λ max = 322 nm ε = 24790
Absorption
1,4
1,2
1,0
0,8
0,6
0,4
0,2
0,0
200
250
300
350
400
λ (nm)

Fig. 2:

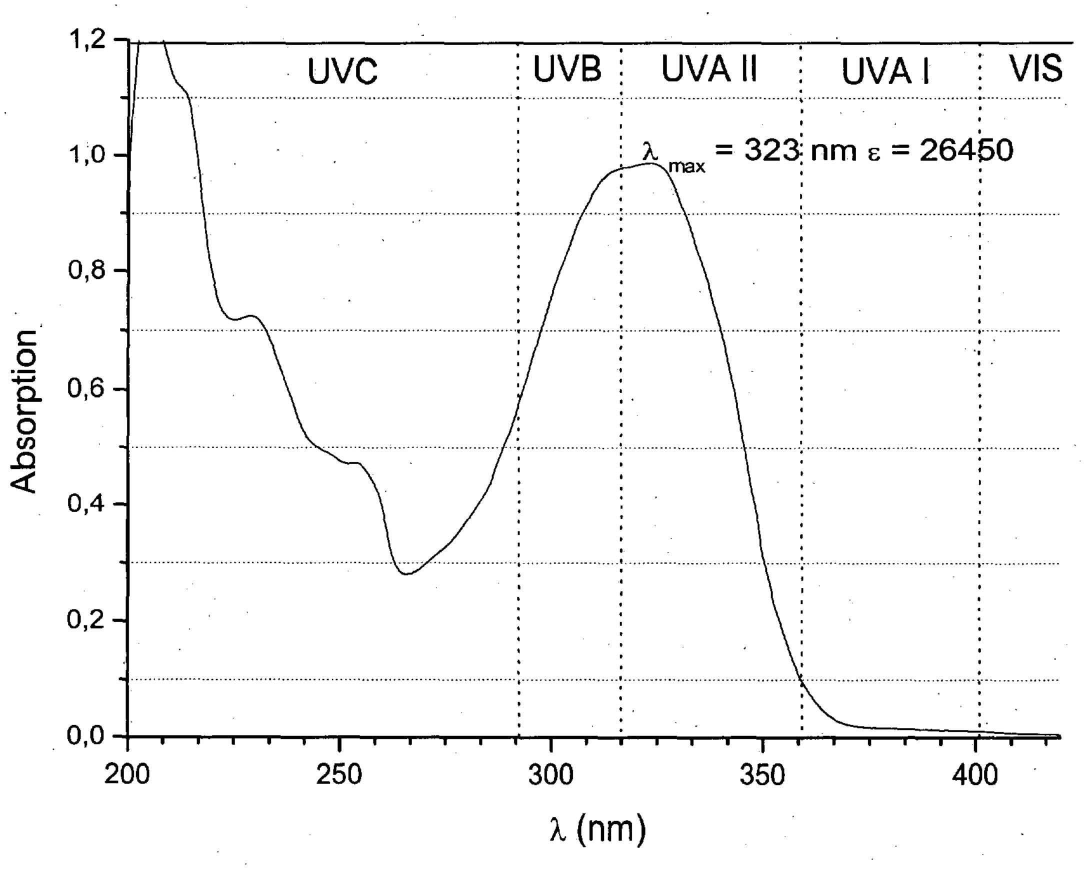
1,2
1,0
0,8
0,6
0,4
0,2
0,0
Absorption
UVC
UVB
UVA II
UVA I
VIS
λ max = 323 nm ε = 26450
200
250
300
350
400
λ (nm)

Fig. 3:

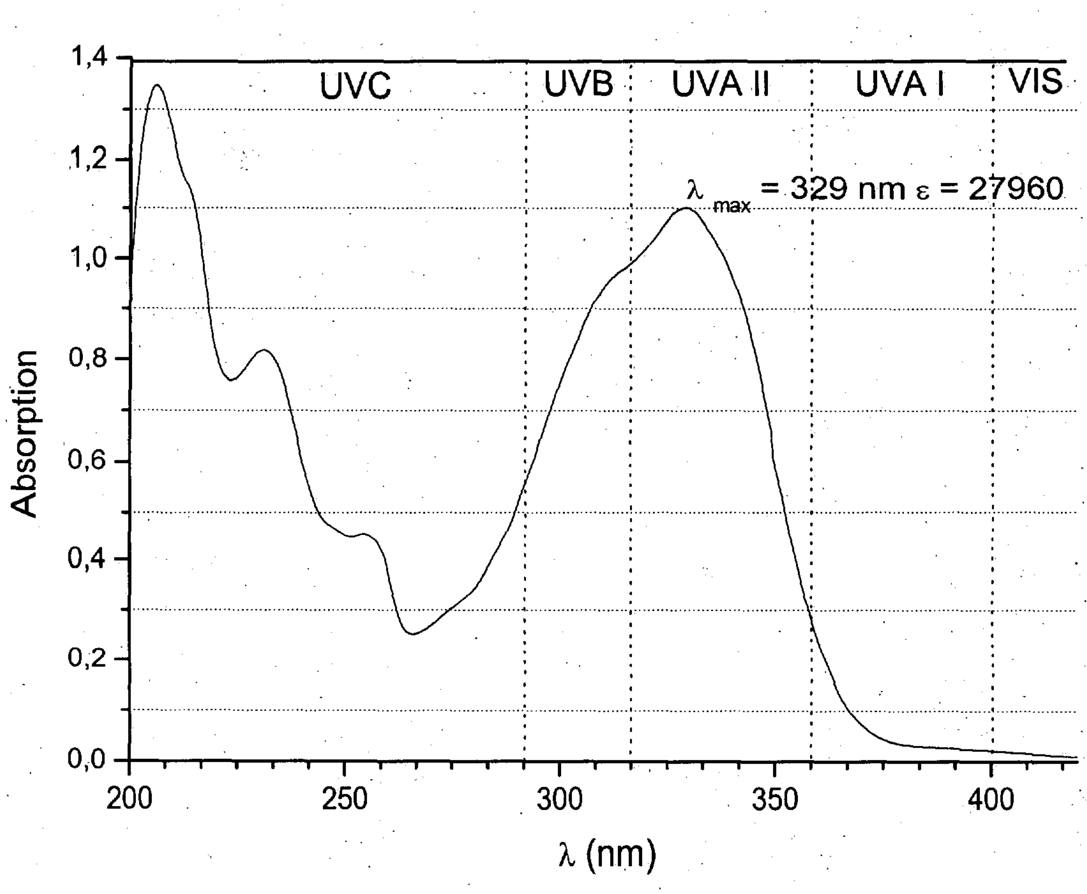
UVC
UVB
UVA II
UVA I
VIS
λ max = 329 nm ε = 27960
Absorption
1,4
1,2
1,0
0,8
0,6
0,4
0,2
0,0
200
250
300
350
400
λ (nm)

Fig. 4:

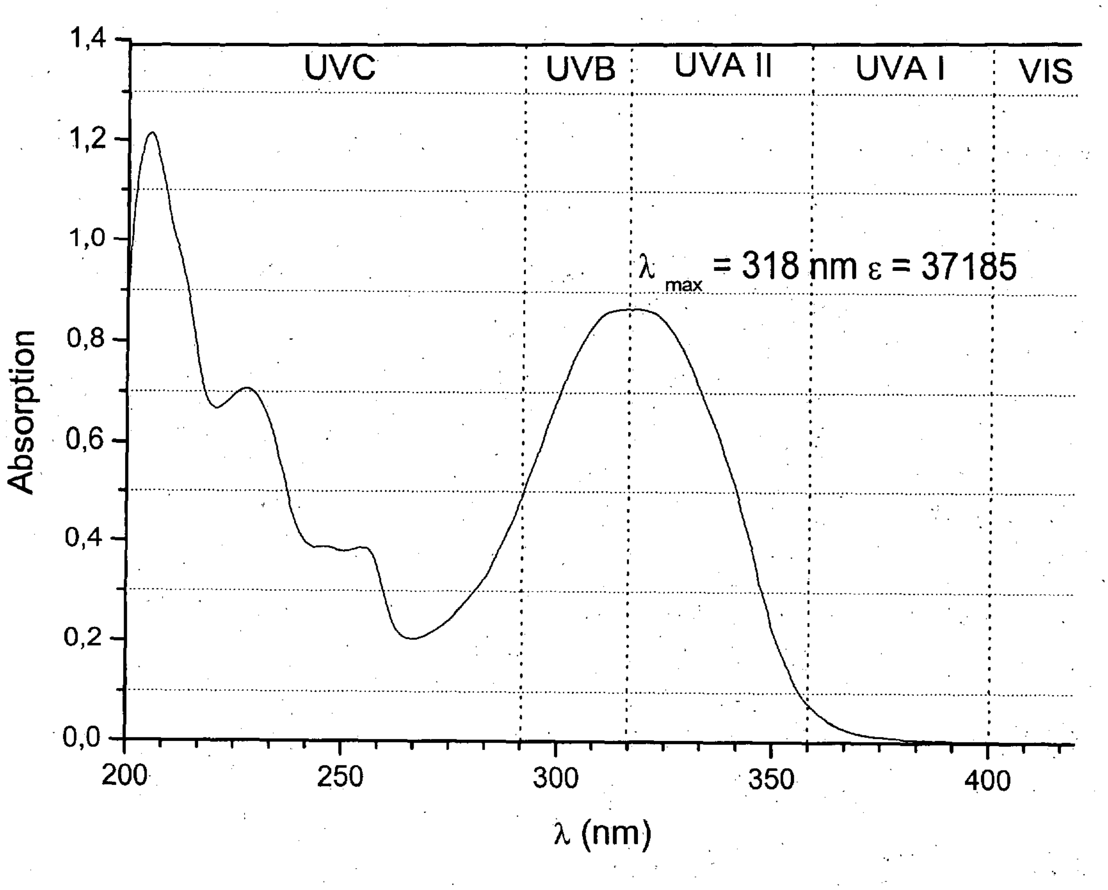
1,4
1,2
1,0
0,8
0,6
0,4
0,2
0,0
Absorption
UVC
UVB
UVA II
UVA I
VIS
λ max = 318 nm ε = 37185
200
250
300
350
400
λ (nm)

Europäisches Patentamt

# EUROPÄISCHER RECHERCHENBERICHT

Nummer der Anmeldung
EP 03 01 3429

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | COTELLE, NICOLE ET AL: "Antioxidant properties of hydroxy-flavones" FREE RADICAL BIOLOGY & MEDICINE (1996), 20(1), 35-43 , XP001027007 * Beispiele 7-10; Tabelle 1 * --- | 1-6,8,9, 11 | A61K7/48 A61K7/42 A61K31/352 A61K31/7048 A61P17/00 A61P39/06 A23L1/30 |
| X,D | DE 197 55 504 A (BEIERSDORF AG) 17. Juni 1999 (1999-06-17) * Ansprüche 1,2,4-7 * * Seite 5, Zeile 46 - Seite 6, Zeile 23 * --- | 1-7,9, 11-13 | |
| X,D | WO 02 00214 A (KRUTMANN JEAN ;STEGE HELGER (DE)) 3. Januar 2002 (2002-01-03) * Anspruch 1 * * Seite 5, Absätze 2,3 * --- | 1-5,8,11 | |
| X | EP 0 124 393 A (MULLER ALBAN INT) 7. November 1984 (1984-11-07) * Ansprüche 1,4 * --- | 1-5,9,11 | RECHERCHIERTE SACHGEBIETE (Int.Cl.7) |
| X | US 5 280 024 A (ROLLAND YVES ET AL) 18. Januar 1994 (1994-01-18) * Ansprüche 1-3,14 * * Beispiele 1,2,13,21 * * Spalte 5, Zeile 1-50 * --- | 1-3,5,8, 9,11 | A61K A61P A23L |
| X | US 5 889 003 A (LEWIN GUY ET AL) 30. März 1999 (1999-03-30) * Ansprüche 1-3,10,11 * * Spalte 11; Beispiel B * * Spalte 9, Zeile 20-34 * --- | 1-3,5,8, 9,11 | |
| X | WO 01 03681 A (PRENDERGAST PATRICK T) 18. Januar 2001 (2001-01-18) * Ansprüche 1,6 * * Seite 25, Zeile 3 - Seite 26, Zeile 4 * * Seite 37, Zeile 9 - Seite 42, Zeile 26 * --- | 1-5,8,9, 11 | |
| | -/-- | | |

Der vorliegende Recherchenbericht wurde für alle Patentansprüche erstellt

| Recherchenort | Abschlußdatum der Recherche | Prüfer |
|---|---|---|
| MÜNCHEN | 26. November 2003 | Hauss, R |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 03 01 3429

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X,P | DATABASE WPI<br>Derwent Publications Ltd., London, GB;<br>AN 2003-375648<br>XP002262815<br>-& JP 2002 255778 A (OGAWA & CO., LTD.),<br>11. September 2002 (2002-09-11)<br>* Zusammenfassung * | 1-5,9,<br>11,12 | |
| X,P,<br>D | WO 02 060889 A (MERCK PATENT GMBH<br>;BUCHHOLZ HERWIG (DE); PERRUCHON SOPHIE<br>(DE)) 8. August 2002 (2002-08-08)<br>* Ansprüche *<br>* Beispiele 2,5 *<br>* Seite 6, Zeile 8-29 * | 1-5,9-11 | |
| E | WO 03 057210 A (KIMBERLY CLARK CO)<br>17. Juli 2003 (2003-07-17)<br>* Ansprüche *<br>* Seite 3, Zeile 18-26 *<br>* Seite 7, Zeile 13 - Seite 9, Zeile 10 * | 1-5,8,9,<br>11 | |

RECHERCHIERTE SACHGEBIETE (Int.Cl.7)

Der vorliegende Recherchenbericht wurde für alle Patentansprüche erstellt

| Recherchenort | Abschlußdatum der Recherche | Prüfer |
|---|---|---|
| MÜNCHEN | 26. November 2003 | Hauss, R |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 03 01 3429

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

26-11-2003

| Im Recherchenbericht angeführtes Patentdokument | Datum der Veröffentlichung | Mitglied(er) der Patentfamilie | Datum der Veröffentlichung |
|---|---|---|---|
| DE 19755504 A | 17-06-1999 | DE 19755504 A1 | 17-06-1999 |
| | | DE 29724644 U1 | 22-08-2002 |
| | | US 5952391 A | 14-09-1999 |
| WO 0200214 A | 03-01-2002 | DE 10031457 A1 | 17-01-2002 |
| | | AU 7234901 A | 08-01-2002 |
| | | WO 0200214 A1 | 03-01-2002 |
| | | EP 1294374 A1 | 26-03-2003 |
| EP 0124393 A | 07-11-1984 | FR 2543434 A1 | 05-10-1984 |
| | | EP 0124393 A1 | 07-11-1984 |
| | | JP 59184117 A | 19-10-1984 |
| US 5280024 A | 18-01-1994 | FR 2689127 A1 | 01-10-1993 |
| | | AT 153022 T | 15-05-1997 |
| | | AU 3559193 A | 07-10-1993 |
| | | CA 2092961 A1 | 01-10-1993 |
| | | DE 69310602 D1 | 19-06-1997 |
| | | DE 69310602 T2 | 18-12-1997 |
| | | DK 564350 T3 | 15-12-1997 |
| | | EP 0564350 A1 | 06-10-1993 |
| | | ES 2104090 T3 | 01-10-1997 |
| | | GR 3023938 T3 | 30-09-1997 |
| | | JP 2082884 C | 23-08-1996 |
| | | JP 6025212 A | 01-02-1994 |
| | | JP 7119221 B | 20-12-1995 |
| | | NZ 247287 A | 26-08-1994 |
| | | US 5457103 A | 10-10-1995 |
| | | ZA 9302312 A | 18-10-1993 |
| US 5889003 A | 30-03-1999 | FR 2753969 A1 | 03-04-1998 |
| | | AT 227712 T | 15-11-2002 |
| | | AU 728990 B2 | 25-01-2001 |
| | | AU 3927197 A | 02-04-1998 |
| | | BR 9704900 A | 27-10-1998 |
| | | CA 2216617 A1 | 27-03-1998 |
| | | CN 1179422 A ,B | 22-04-1998 |
| | | DE 69717044 D1 | 19-12-2002 |
| | | DE 69717044 T2 | 24-07-2003 |
| | | DK 832886 T3 | 10-03-2003 |
| | | EP 0832886 A1 | 01-04-1998 |
| | | ES 2187738 T3 | 16-06-2003 |
| | | HU 9701588 A2 | 28-12-1998 |
| | | JP 10114766 A | 06-05-1998 |
| | | NO 974455 A | 30-03-1998 |
| | | NZ 328858 A | 26-01-1998 |

EPO FORM P0461

Für nähere Einzelheiten zu diesem Anhang : siehe Amtsblatt des Europäischen Patentamts, Nr.12/82

## ANHANG ZUM EUROPÄISCHEN RECHERCHENBERICHT ÜBER DIE EUROPÄISCHE PATENTANMELDUNG NR. EP 03 01 3429

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

26-11-2003

| Im Recherchenbericht angeführtes Patentdokument | Datum der Veröffentlichung | Mitglied(er) der Patentfamilie | Datum der Veröffentlichung |
|---|---|---|---|
| US 5889003 A | | PL 322297 A1 | 30-03-1998 |
| | | PT 832886 T | 31-03-2003 |
| | | ZA 9708652 A | 27-03-1998 |
| WO 0103681 A | 18-01-2001 | AU 6174900 A | 30-01-2001 |
| | | CA 2375647 A1 | 18-01-2001 |
| | | EP 1223928 A2 | 24-07-2002 |
| | | WO 0103681 A2 | 18-01-2001 |
| | | JP 2003504327 T | 04-02-2003 |
| | | US 6555523 B1 | 29-04-2003 |
| JP 2002255778 A | 11-09-2002 | KEINE | |
| WO 02060889 A | 08-08-2002 | DE 10104350 A1 | 14-08-2002 |
| | | WO 02060889 A1 | 08-08-2002 |
| | | EP 1355898 A1 | 29-10-2003 |
| WO 03057210 A | 17-07-2003 | US 2003125264 A1 | 03-07-2003 |
| | | WO 03057210 A1 | 17-07-2003 |

EPO FORM P0461

Für nähere Einzelheiten zu diesem Anhang : siehe Amtsblatt des Europäischen Patentamts, Nr.12/82